# EUROPEAN PATENT SPECIFICATION

(11) **EP 1 455 593 B1**
(45) Date of publication and mention of the grant of the patent: **24.07.2013**
(21) Application number: 02782843.3
(22) Date of filing: 07.10.2002
(51) Int. Cl.: A23K 1/16, A23K 1/18

(54) **METHODS AND COMPOSITIONS FOR PROMOTING GROWTH AND INNATE IMMUNITY IN YOUNG ANIMALS**
VERFAHREN UND ZUSAMMENSETZUNGEN UM WACHSTUM UND KONGENITALE IMMUNITÄT VON JUNGEN TIEREN ZU FÖRDERN
PROCEDES ET COMPOSITIONS POUR FAVORISER LA CROISSANCE ET L'IMMUNITE NATURELLE CHEZ DE JEUNES ANIMAUX

(30) Priority: 06.10.2001 US 327703 P
(43) Date of publication of application: 15.09.2004
(73) Proprietor: Merial Limited, Duluth, GA 30096 (US); UNIVERSITY OF SASKATCHEWAN, Saskatoon, Saskatchewan S7N 5C8 (CA)
(72) Inventor: BABIUK, Lorne, Alan, Saskatoon, Saskatchewan S7J 2Y1 (CA); HECKER, Rolf, 40597 Düsseldorf (DE); GRIEBEL, Philip, J., Saskatoon, Saskatchewan S7K 3J8 (CA); POTTER, Andrew, Allan, Saskatoon, Saskatchewan F7H 3S5 (CA); GOMIS, Susantha, Saskatoon, Saskatchewan S7N 4A7 (CA)
(74) Representative: Harding, Charles Thomas
(86) International application number: PCT/EP2002/011212
(87) International publication number: WO 2003/030656

(56) References cited:
- EP-A- 0 107 161
- WO-A-01/22972
- WO-A-99/56755
- WO-A1-01/89316
- DE-A- 10 024 746
- WPI WORLD PATENT INFORMATION DERWENT, DERWENT, GB, vol. 1995, no. 38 PA - NIPPON SEISHI KK PN - JP7184595 A, 25 July 1995 (1995-07-25), XP002158862
- KUBITZA F ET AL: "IDENTIFICATION OF FEED ENHANCERS FOR JUVENILE LARGEMOUTH BASS MICROPTERUS SALMOIDES" AQUACULTURE, AMSTERDAM, NL, vol. 148, no. 2/3, 1997, pages 191-200, XP000979269

## Description

### Field

The description relates to methods and compositions for inducing weight growth and innate immunity in young animals, including neonates, using immunostimulatory nucleic acids.

### Background of the Invention

Agricultural livestock and in some cases zoo animals are ripe targets for widespread transmission of pathogenic diseases for two major reasons.. First, livestock usually live in such close quarters thereby facilitating the transmission of an infectious pathogen to an entire flock or herd. Second, because many infectious pathogen eventually exit the body in feces which invariably litter a grazing field for animals, the likelihood of transmission and widespread infection is high. Thus, the ability to prevent a wide variety of infections in such animals would be highly desirable in these circumstances.

One of the other major obstacles to increasing the productivity of livestock operations (e.g., livestock farming) is the time required to bring livestock to a suitable weight for slaughter and processing purposes. The ability to decrease the time required for an animal to achieve the minimum weight for slaughter would be highly desirable in the agricultural industry as it would increase productivity and profitability.

WO9956755 discloses a method for preventing parasitic infections and infection related diseases by the administration of a CpG oligonucleotide prior to parasite exposure to a subject at risk of parasite exposure.

EP0107161 discloses the use of active compounds to optimize the tissue mass of organs within the range of genetic variation of humans and animals wherein the active compounds are oligonucleotides, the terminal bases of which differ from adenine, and/or nucleic acid bases with the exception of adenine, and/or carboxyl derivative of nucleic acid bases with the exception of adenine, and/or amino derivatives of nucleic acid bases, with the exception of adenine.

JP7184595 discloses a yeast extract composition having an immune-enhancing action and an intake-stimulating action and suitable for feeds.

Kubitza et al 1997 (Aquaculture 148: 191-200) disclose trials to identify feed enhancers and that inosina-5-monophospahte and fish meal are effective feed enhancers.

WO01/89315 discloses a feedstuff additive made from plant material and the use thereof for feeding animals.

### Summary of the Invention

The description relates broadly to the use of immunostimulatory nucleic acids for promoting overall growth and innate immunity within non-human animals.

The present invention relates to the use of an immunostimulatory nucleic acid in the manufacture of a composition for preventing infection in feed animals; wherein said immunostimulatory nucleic acid is to be administered subcutaneously in the abdominal area; and wherein the immunostimulatory nucleic acid is a CpG oligonucleotide or a T-rich oligonucleotide or a poly-G oligonucleotide or a methylated CpG oligonucleotide; wherein the immunostimulatory nucleic acid increases the rate of growth of a feed animal; wherein said immunostimulatory nucleic acid is in an effective amount to increase the rate of growth of the feed animal; and wherein the feed animal is a chicken.

In another aspect, the present invention relates to a composition comprising an immunostimulatory nucleic acid for use in preventing infection in feed animals wherein said immunostimulatory nucleic acid is to be administered subcutaneously in the abdominal area; and wherein the immunostimulatory nucleic acid is a CpG oligonucleotide or a T-rich oligonucleotide or a poly-G oligonucleotide or a methylated CpG oligonucleotide; wherein the immunostimulatory nucleic acid increases the rate of growth of a feed animal; wherein said immunostimulatory nucleic acid is in an effective amount to increase the rate of growth of the feed animal; and wherein the feed animal is a chicken.

In a further aspect, the present invention relates to the use of an immunostimulatory nucleic acid in the preparation of a composition for preventing infection in feed animals by promoting innate immunity in a non-human animal; wherein said immunostimulatory nucleic acid is to be administered subcutaneously in the abdominal area without the co-administration of an antigen; wherein the immunostimulatory nucleic acid is a CpG oligonucleotide or a T-rich oligonucleotide or a poly-G oligonucleotide or a methylated CpG oligonucleotide; and wherein the non-human animal is at an age which is less than 10% of the average life span of said non-human animal; and wherein the non-human animal is a chicken.

The present invention relates to, in another aspect, a composition comprising an immunostimulatory nucleic acid for use in preventing infection in feed animals by promoting innate immunity in a non-human animal; wherein said immunostimulatory nucleic acid is to be administered subcutaneously in the abdominal area without the co-administration of an antigen; wherein the immunostimulatory nucleic acid is a CpG oligonucleotide or a T-rich oligonueleotide or a poly-G oligonucleotide or a methylated CpO oligonucleotide; and wherein the non-human animal is at an age which is less than 10% of the average life span of said non-human animal; and wherein the non-human animal is a chicken.

In one aspect, the description provides a method for increasing the rate of growth of feed animals. The method comprises administering to a feed animal an immunostimulatory nucleic acid in an effective amount to increase the rate of growth of the feed animal. In one embodiment, the immunostimulatory nucleic acid is administered at regular intervals. In a related embodiments, the immunostimulatory nucleic acid is administered on a weekly basis, on a daily basis, or a monthly basis. The immunostimulatory nucleic acid may be administered with animal feed.

In one embodiment, the immunostimulatory nucleic acid is administered to a feed animal within a week of birth. In another embodiment, the immunostimulatory nucleic acid is administered to a feed animal within a day of birth. In yet a further embodiment, the immunostimulatory nucleic acid is administered to a feed animal immediately after birth. The immunostimulatory nucleic acid may also be administered to a pregnant feed animal.

The immunostimulatory nucleic acid may be administered orally, or by injection, but administration is not so limited. In some embodiment, the immunostimulatory nucleic acid is administered through a sustained release device. In some embodiments, the immunostimulatory nucleic acid is administered in an absolute dosage range of 5 *µ*g to 250 *µ*g, preferably 5 *µ*g to 100 *µ*g, and more preferably 5 *µ*g to 50 *µ*g.

The feed animals may be cows, pigs, buffalo, chicken, ducks, pigeons, turkeys, geese, rabbits, deer, goats, sheep, quail, bison, horse, moose, and shellfish such as shrimp, lobster, clams, oysters and mussels. In the invention, the feed animal is a chicken.

In some important embodiments, the time required for the chicken to reach acceptable size for feed is reduced by one day compared to a chicken that is not administered the immunostimulatory nucleic acid. In other important embodiments, the time required for the chicken to reach acceptable size for feed is reduced by three days compared to a chicken that is not administered the immunostimulatory nucleic acid.

The young non-human animal may be one that was born prematurely. The young non-human animal is a multiple delivery animal.

A nucleic acid is an element each aspect of the description. The nucleic acids useful according to the description may be synthetic or natural nucleic acids. In some preferred embodiments, the nucleic acids are isolated or substantially purified.

Generally the nucleic acids are immunostimulatory nucleic acids. The immunostimulatory nucleic acid is any nucleic acid which is capable of modulating an immune response. However, in some embodiments, the immunostimulatory nucleic acid is administered in an amount that will promote growth of the subject rather than induce an immune response. In some embodiments, the immunostimulatory nucleic acid is a CpG nucleic acid having an unmethylated CpG motif (particularly an unmethylated C in a CpG dinucleotide), a T-rich nucleic acid (including a poly T nucleic acid), a poly G nucleic acid, or a methylated CpG nucleic acid having a methylated CpG motif.

The nucleic acid, in some embodiments, has a nucleotide backbone which includes at least one backbone modification, such as a phosphorothioate modification or other phosphate modification. In other embodiments, the immunostimulatory nucleic acids are "non-motif' phosphorothioate nucleic acids which are defined herein as nucleic acids having at least one phosphorothioate backbone modification and lacking an immunostimulatory motif selected from the group of unmethylated CpG motif, a methylated CpG motif, T-rich motif and poly G motif. In some embodiments, the modified backbone is a peptide modified oligonucleotide backbone. The nucleotide backbone may be chimeric, or the nucleotide backbone may be entirely modified.

The immunostimulatory nucleic acid can have any length greater than 6 nucleotides, but, in some embodiments, is between 8 and 100 nucleotide residues in length. In another embodiment, the nucleic acid may be between 8 and 40 nucleotides in length. In other embodiments, the nucleic acid comprises at least 20 nucleotides, at least 24 nucleotides, at least 27 nucleotides, or at least 30 nucleotides. The nucleic acid may be single stranded or double stranded. In some embodiments, the nucleic acid is isolated and in other embodiments, the nucleic acid may be a synthetic nucleic acid.

In one embodiment, the immunostimulatory nucleic acid is a CpG immunostimulatory nucleic acid (also referred to herein as a CpG nucleic acid). The CpG nucleic acid, in one embodiment, contains at least one unmethylated CpG dinucleotide having a sequence including at least the following formula: 5' X₁ X₂CGX₃ X₄ 3' wherein C is unmethylated, wherein X₁, X₂, X₃, and X₄ are nucleotides. In another embodiment, the methylated CpG nucleic acid comprises: 5' X₁ X₂CGX₃ X₄ 3' wherein C is methylated, wherein X₁, X₂, X₃, and X₄ are nucleotides. In one embodiment, the 5' X₁ X₂CGX₃ X₄ 3' sequence of the CpG nucleic acid or the methylated CpG nucleic acid is a non-palindromic sequence, and in other embodiments, it is a palindromic sequence.

In some embodiments, X₁X₂ are nucleotides selected from the group consisting of: GpT, GpG, GpA, ApA, ApT, ApG, CpT, CpA, CpG, TpA, TpT, and TpG; and X₃X₄ are nucleotides selected from the group consisting of: TpT, CpT, ApT, TpG, ApG, CpG, TpC, ApC, CpC, TpA, ApA, and CpA. In other embodiments, X₁X₂ are GpA or GpT and X₃X₄ are TpT. In yet other embodiments, X₁ or X₂ or both are purines and X₃ or X₄ or both are pyrimidines or X₁X₂ are GpA and X₃ or X₄ or both are pyrimidines. In one embodiment, X₂ is a T and X₃ is a pyrimidine.

In some embodiments, the CpG nucleic acid is selected from the group consisting of TCGTCGTTGTCGTTTGTCGTT; TCGCGTGCGTTTTGTCGTTTTGACGTT; TCGTCGTTTGTCGTTTTGTCGTT); and ggGGGACGATCGTCgggggG. In the last sequence, lower case G's represent residues that are linked together by phosphorothioate linkages, while upper case G's represent residues that are linked together by phosphodiester linkages.

Other examples of CpG nucleic acids suitable for use in the present description are described in published PCT application PCT/US00/26383 (International Publication Number WO 01/22972), published on April 5, 2001.

In other embodiments, the immunostimulatory nucleic acid is a T-rich immunostimulatory nucleic acid (also referred to herein as a T-rich nucleic acid). In some embodiments, the T-rich immunostimulatory nucleic acid is a poly T nucleic acid comprising 5' TTTT 3'. In yet other embodiments, the poly T nucleic acid comprises 5' X₁ X₂TTTTX₃ X₄ 3' wherein X₁, X₂, X₃ and X₄ are nucleotides. In some embodiments, X₁X₂ is TT and/or X₃X₄ is TT. In other embodiments, X₁X₂ is selected from the group consisting of TA, TG, TC, AT, AA, AG, AC, CT, CC, CA, CG, GT, GG, GA, and GC; and/or X₃X₄ is selected from the group consisting of TA, TG, TC, AT, AA, AG, AC, CT, CC, CA, CG, GT, GG, GA, and GC.

The T-rich immunostimulatory nucleic acid may have only a single poly T motif or it may have a plurality of poly T nucleic acid motifs. In some embodiments, the T-rich immunostimulatory nucleic acid comprises at least 2, at least 3, at least 4, at least 5, at least 6, at least 7, or at least 8 poly T motifs. In other embodiments, it comprises at least 2, at least 3, at least 4, at least 5, at least 6, at least 7, or at least 8 CpG motifs. In some embodiments, the plurality of CpG motifs and poly T motifs are interspersed.

In yet other embodiments, at least one of the plurality of poly T motifs comprises at least 3, at least 4, at least 5, at least 6, at least 7, at least 8, or at least 9 contiguous T nucleotide residues. In other embodiments, the plurality of poly T motifs is at least 3 motifs and wherein at least 3 motifs each comprises at least 3 contiguous T nucleotide residues or the plurality of poly T motifs is at least 4 motifs and wherein the at least 4 motifs each comprises at least 3 contiguous T nucleotide residues.

The T-rich immunostimulatory nucleic acid may include one or more CpG motifs. The motifs may be methylated or unmethylated. In other embodiments, the T-rich immunostimulatory nucleic acid is free of one or more CpG dinucleotides.

In other embodiments, the T-rich immunostimulatory nucleic acid has poly A, poly G, and/or poly C motifs. In other embodiments, the T-rich immunostimulatory nucleic acid is free of two poly C sequences of at least 3 contiguous C nucleotide residues. Preferably the T-rich immunostimulatory nucleic acid is free of two poly A sequences of at least 3 contiguous A nucleotide residues. In other embodiments, the T-rich immunostimulatory nucleic acid comprises a nucleotide composition of greater than 25% C or greater than 25% A. In yet other embodiments, the T-rich immunostimulatory nucleic acid is free of poly C sequences, poly G sequences or poly-A sequences.

In some cases the T-rich immunostimulatory nucleic acid may be free of poly T motifs, but rather, may comprise a nucleotide composition of greater than 25% T. In other embodiments, the T-rich immunostimulatory nucleic acid may have poly T motifs and may also comprise a nucleotide composition of greater than 25% T. In some embodiments, the T-rich immunostimulatory nucleic acid comprises a nucleotide composition of greater than 25% T, greater than 30% T, greater than 40% T, greater than 50% T, greater than 60% T, greater than 80% T, or greater than 90% T nucleotide residues. In other embodiments, the T-rich nucleic acids are at least 20 nucleotides in length or at least 24 nucleotides in length.

Examples of T rich nucleic acids that are free of CpG nucleic acids and of T rich nucleic acids that include CpG nucleic acids are described in published PCT application PCT/US00/26383 (International Publication Number WO 01/22972), published on April 5, 2001.

In some embodiments, the poly G nucleic acid comprises: 5' X₁X₂GGGX₃X₄ 3' wherein X_{1.} X_{2.} X_{3.} and X₄ are nucleotides. In other embodiments, at least one of X₃ and X₄ are a G or both of X₃ and X₄ are a G. In still other embodiments, the poly G nucleic acid comprises the following formula: 5' GGGNGGG 3' wherein N represents between 0 and 20 nucleotides. In yet other embodiments, the poly G nucleic acid comprises the following formula: 5' GGGNGGGNGGG 3' wherein N represents between 0 and 20 nucleotides.

The poly G immunostimulatory nucleic acid may include one or more CpG motifs or T-rich motifs. The CpG motifs may be methylated or unmethylated. The poly G nucleic acid may include at least one unmethylated CpG dinucleotide. In other embodiments, the poly G nucleic acid is free of one or more CpG dinucleotides or T-rich motifs.

In some embodiments, the poly G nucleic acid is free of unmethylated CG dinucleotides. In other embodiments, the poly G nucleic acid includes at least one unmethylated CG dinucleotide. Examples of both types are described in published PCT application PCT/US00/26383 (International Publication Number WO 01/22972), published on April 5, 2001.

The nucleic acid may be administered alone or in conjunction with a pharmaceutically-acceptable carrier and optionally other therapeutic agents. Other therapeutic agents include, but are not limited to other growth promotants (such as bovine growth hormone), and other innate immunity inducers.

In another aspect, the description provides a composition comprising an immunostimulatory nucleic acid formulated for oral administration to a feed animal. Preferably, the immunostimulatory nucleic acid is formulated in an amount effective for promoting weight growth. In important embodiments, the composition does not further comprise an antigen.

In some preferred embodiments, the composition further comprises an animal feed (or is formulated as part of an animal feed). The animal feed may be selected from the group consisting of chicken feed, cattle feed, hog feed, fish feed, and rabbit feed, but is not so limited.

In yet another aspect, the description provides a composition comprising an immunostimulatory nucleic acid formulated for subcutaneous administration to a feed animal. In one embodiment, the immunostimulatory nucleic acid is formulated in a sustained release vehicle for subcutaneous administration.

In still another aspect, the description provides a method for promoting innate immunity in a young non-human animal. The method comprises administering to a young non-human animal a immunostimulatory nucleic acid without the co-administration of an antigen in order to induce innate immunity.

In one embodiment, the immunostimulatory nucleic acid is administered at regular intervals. The immunostimulatory nucleic acid may be administered with animal feed, but its method of administration is not so limited. In some important embodiments, immunostimulatory nucleic acid is administered subcutaneously.

In one embodiment, the immunostimulatory nucleic acid is administered within a week of birth. In another embodiment, the immunostimulatory nucleic acid is administered within a day of birth. In yet another embodiment, the immunostimulatory nucleic acid is administered immediately after birth. The administration of the immunostimulatory nucleic acid may be continued as the animal ages. Alternatively, the administration may be discontinued as the animal ages.

The young non-human animal may be one that was born prematurely. The young non-human animal is a multiple delivery animal.

In the invention the non-human animal is a chicken. The immunostimulatory nucleic acid is preferably administered at an absolute dose of at least 5 *µ*g. The immunostimulatory nucleic acid may be administered in an absolute dose range of 5 *µ*g to 250 *µ*g. In other embodiments, the immunostimulatory nucleic acid is administered in an absolute dose range of 5 *µ*g to 50 *µ*g. And in still further embodiments, the immunostimulatory nucleic acid is administered at an absolute dose of 50 *µ*g.

In yet a further aspect, the description provides a method for promoting innate immunity in a non-human animal in utero. The method comprises administering to a non-human animal in utero an immunostimulatory nucleic acid without the co-administration of an antigen in order to induce innate immunity.

The immunostimulatory nucleic acid may be administered to a pregnant animal orally. The immunostimulatory nucleic acid may be administered with animal feed. The immunostimulatory nucleic acid may be administered in a lipid based delivery system in an animal feed. The immunostimulatory nucleic acid may be administered in an alginate delivery system in an animal feed. The immunostimulatory nucleic acid may be injected, for example into the pregnant animal or into the amniotic sac. The immunostimulatory nucleic acid may be administered to a pregnant animal trans-placentally or intra-uterine. The immunostimulatory nucleic acid may be administered to a pregnant animal parenterally (e.g., subcutaneously).

The administration of immunostimulatory nucleic acid may be continued after birth. The administration of immunostimulatory nucleic acid may be discontinued after birth.

### Brief Description of the Figures

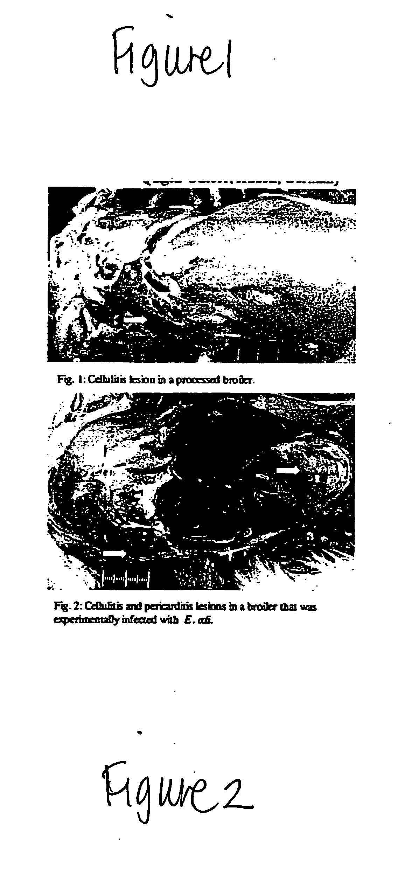

Figure 1 is a digital photograph of a cellulitis lesion in a broiler chicken having been challenged with E. coli.
Figure 2 is a digital photograph of cellulitis and pericarditis lesions in a chicken experimentally infected with E. coli.

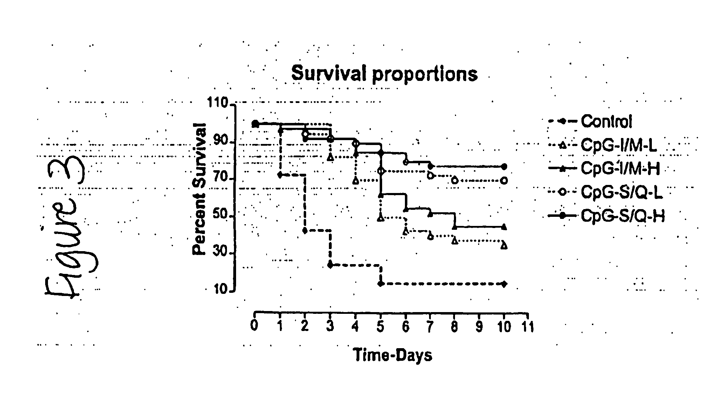

Figure 3 is a survival curve from Experiment 1. Closed circles represent chickens receiving 50 *µ*g CpG nucleic acid administered subcutaneously, open circles represent chickens receiving 10 *µ*g CpG nucleic acid administered subcutaneously, closed triangles represent chickens receiving 50 *µ*g CpG nucleic acid administered intramuscularly, and the open circles represent chickens receiving 10 *µ*g CpG nucleic acid administered intramuscularly. The asterisks represent animals not receiving any CpG nucleic acid. Three days after CpG nucleic acid administration, all animals were exposed to E. coli via a caudal abdominal scratch (0 days on the x-axis). Animals were assessed for morbidity for the next 10 days, at which point any remaining animals were sacrificed.

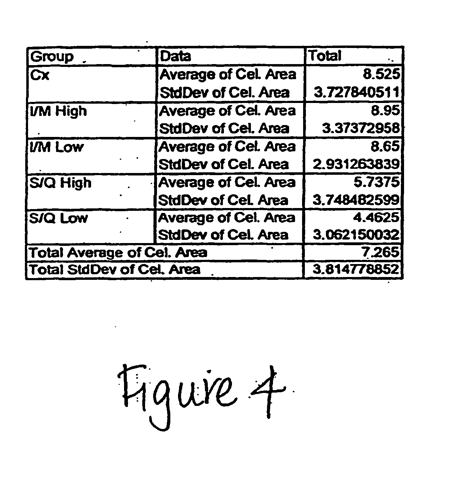

Figure 4 is a table indicating the average size of cellulitis lesions in the chicken groups described in Figure 3. The control (Cx) group and both intramuscular injection groups had similar lesion sizes indicating that intramuscular injection of CpG nucleic acid did not induce local immunity at a distant site of infection. Both subcutaneous injection groups demonstrated smaller cellulitis lesions as compared to the control group. The difference between the control and subcutaneous groups was statistically significant. (Experiment 1)

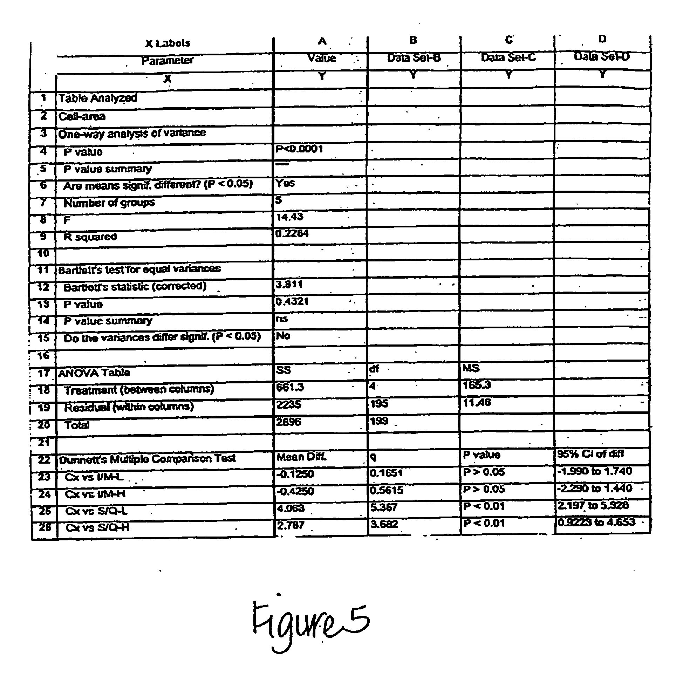

Figure 5 is a table indicating the mean difference in the size of the cellulitis lesions between the groups in Figures 3 and 4. The mean difference in size between the low dose subcutaneous group and the control was 4.063 cm², while the mean difference in size between the high dose subcutaneous group and the control was 2,787 cm². P values are indicated, as are 95% confidence intervals. (Experiment 1)

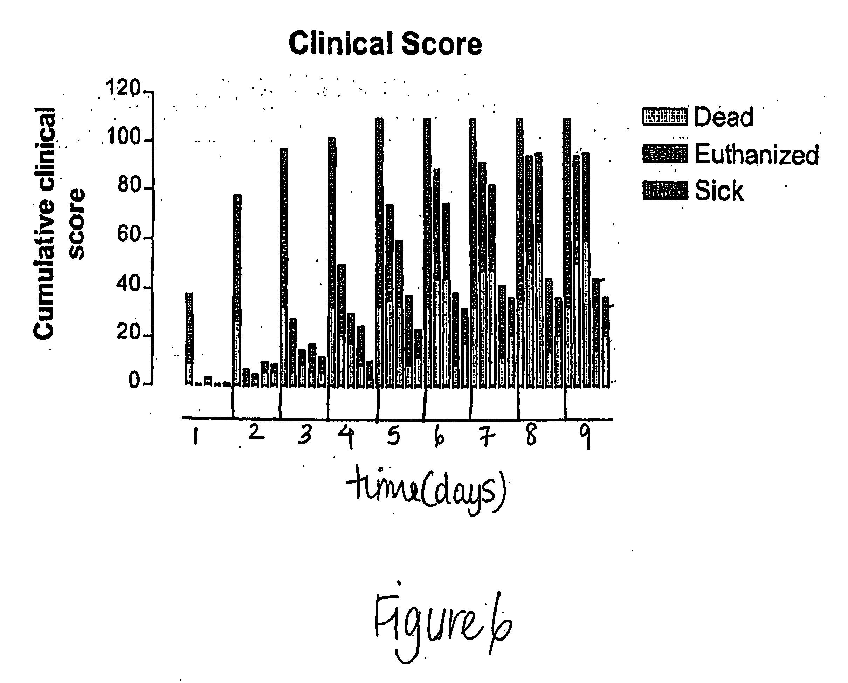

Figure 6 is a bar graph showing cumulative clinical score for the groups in Experiment 1. The x-axis represents time in days. Each day provides data for each group of chickens. The first bar for each day represents the control group, the second bar for each day represent the low dose (10 *µ*g) IM, the third bar represents high dose (50 *µ*g) IM, the fourth bar represents low dose (10*µ*g) SC, and the fifth bar represents high dose (50 *µ*g) SC. Each bar includes data regarding the number of chickens that are sick, the number of chickens that are dead, and those that had to be euthanized because they had achieved predetermined criteria for euthanization. Both subcutaneous injection groups had the fewest sick, dead and euthanized chickens at day 10.

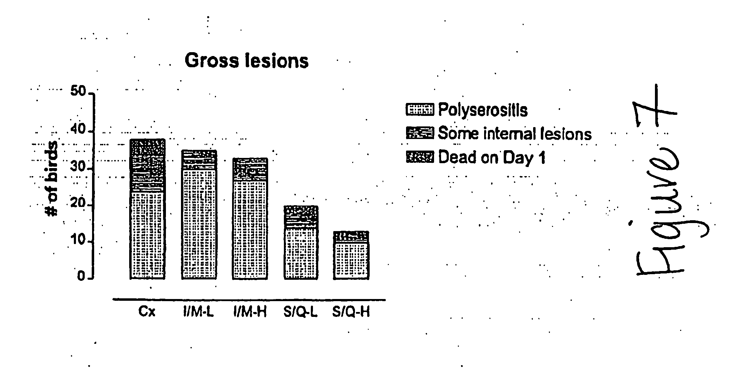

Figure 7 is a bar graph showing the proportion of chickens diagnosed with polyserositis (using predetermined criteria), those diagnosed with some internal lesions, and those dead on day 1. The subcutaneous injected chickens were the least affected group. (Experiment 1)

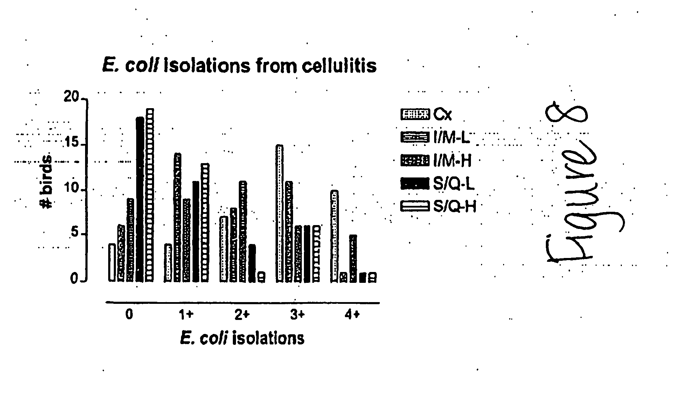

Figure 8 is a bar graph showing the proportion of chickens diagnosed with E. coli isolations from cellulitis lesions. The x-axis represents a grading system based on the number of E. coli isolations in the cellulitis lesions. The legend describes the groups. The data represents the analysis of chickens at various times from day 1 to day 10. Chickens were analyzed at the time of death of euthanization, either from days 1 to 10, or after day 10 at the termination of the experiment. (Experiment 1)

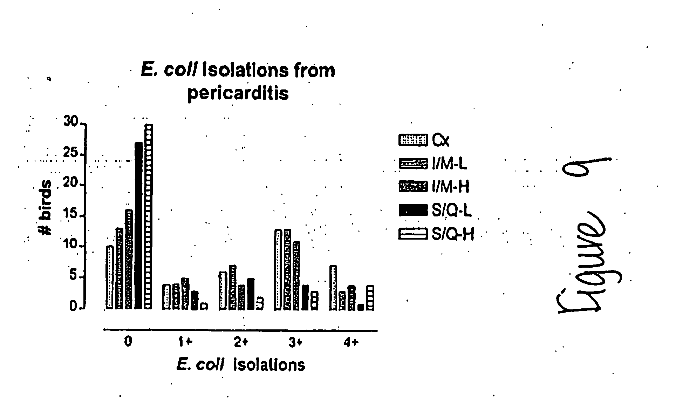

Figure 9 is a bar graph showing the proportion of chickens diagnosed with E. coli isolations from pericarditis lesions. The x-axis represents a grading system based on the number of E. coli isolations in the pericarditis lesions. The legend describes the groups. The data represents the analysis of chickens at various times from day 1 to day 10. Chickens were analyzed at the time of death of euthanization, either from days 1 to 10, or after day 10 at the termination of the experiment. (Experiment 1)

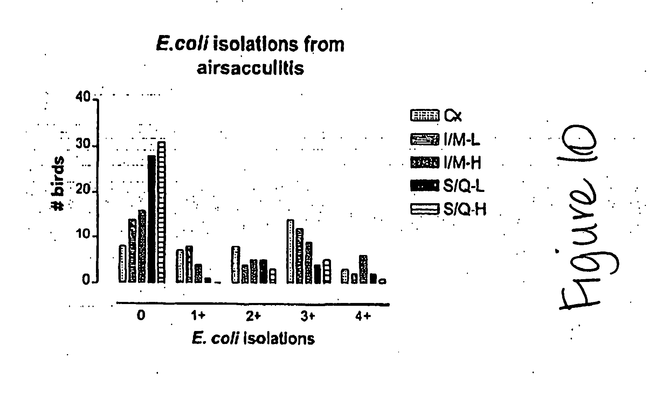

Figure 10 is a bar graph showing the proportion of chickens diagnosed with E. coli isolations from airsacculitis lesions. The x-axis represents a grading system based on the number of E. coli isolations in the airsacculitis lesions. The legend describes the groups. The data represents the analysis of chickens at various times from day 1 to day 10. Chickens were analyzed at the time of death of euthanization, either from days 1 to 10, or after day 10 at the termination of the experiment. (Experiment 1)

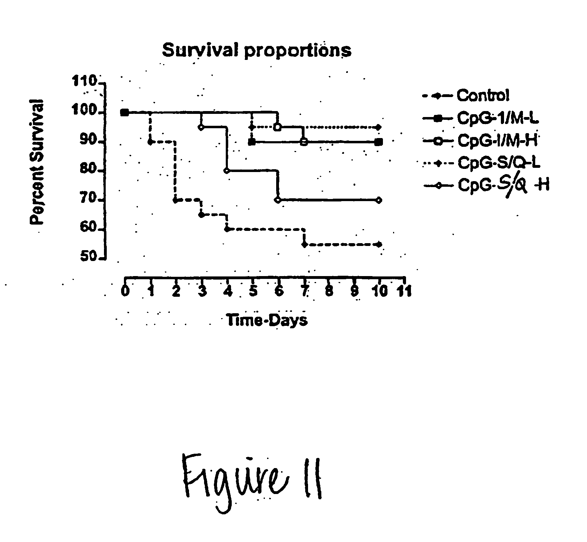

Figure 11 is a survival curve from Experiment 2. Open diamonds represent chickens receiving 50 *µ*g CpG nucleic acid administered subcutaneously, closed diamonds represent chickens receiving 10 *µ*g CpG nucleic acid administered subcutaneously, open squares represent chickens receiving 50 *µ*g CpG nucleic acid administered intramuscularly, and the closed squares represent chickens receiving 10 *µ*g CpG nucleic acid administered intramuscularly. The asterisks represent animals not receiving any CpG nucleic acid. Three days after CpG nucleic acid administration, all animals were exposed to E. coli via a caudal abdominal scratch (0 days on the x-axis). Animals were assessed for morbidity for the next 10 days, at which point any remaining animals were sacrificed.

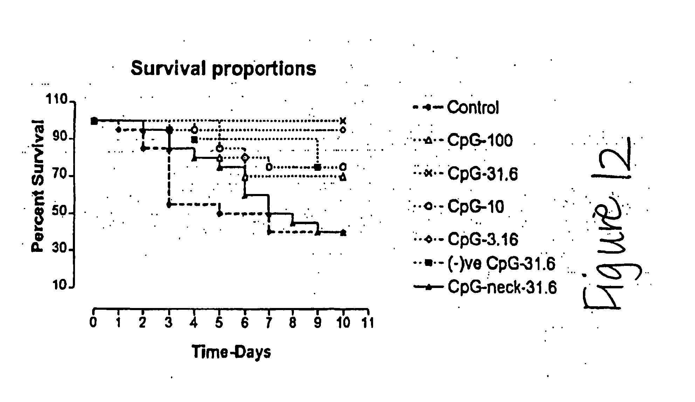

Figure 12 is a survival curve from Experiment 3, a dose titration curve. CpG nucleic acids were administered subcutaneously. Open triangles represent chickens receiving 100 *µ*g CpG nucleic acid, "X" represent chickens receiving 31.6 *µ*g CpG nucleic acid, open circles represent chickens receiving 10 *µ*g CpG nucleic acid, open diamonds represent chickens receiving 3.16 *µ*g CpG nucleic acids, closed squares represent chickens receiving 31.6 *µ*g of a negative control nucleic acids having no identifiable immunostimulatory motif, and the closed triangles represent chickens receiving 31.6 *µ*g CpG nucleic acid subcutaneously in the neck. The asterisks represent animals not receiving any CpG nucleic acid. Three days after CpG nucleic acid administration, all animals were exposed to E. coli via a caudal abdominal scratch (0 days on the x-axis). Animals were assessed for morbidity for the next 10 days, at which point any remaining animals were sacrificed.

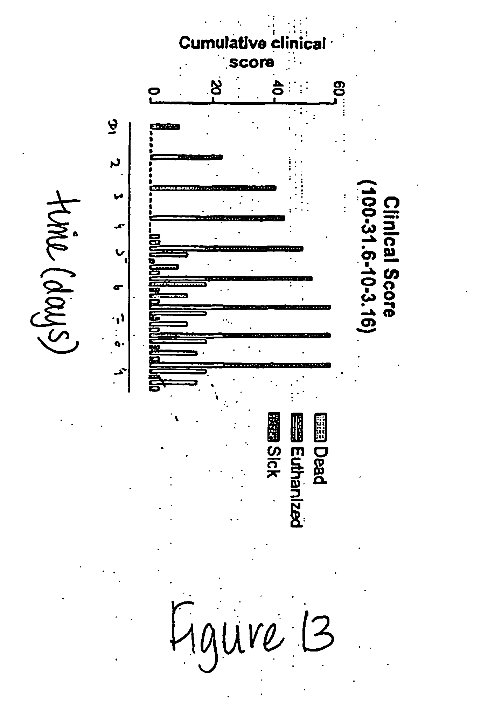

Figure 13 is a bar graph showing the cumulative clinical score of dead, euthanized and sick animals during the course of Experiment 3 (see Figure 12). The first bar of each day represents the control group, the second bar represents the 100 *µ*g CpG nucleic acid group, the third bar represents the 31.6 *µ*g CpG nucleic acid group, the fourth bar represents the 10 *µ*g CpG nucleic acid group, and the fifth group represents the 3.16 *µ*g CpG nucleic acid group. The data if presented for the 10 day course of Experiment 3.

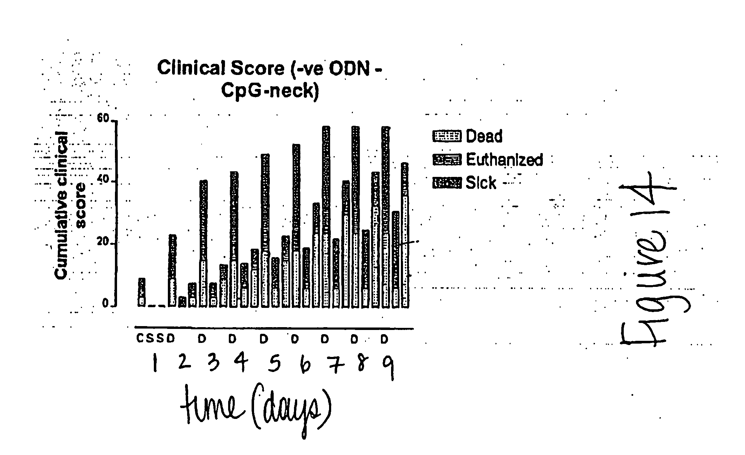

Figure 14 is a bar graph showing the cumulative clinical score of dead, euthanized and sick chickens that received the control nucleic acid or that were administered CpG nucleic acid subcutaneously in the neck. The first bar for each day represents the no nucleic acid control, the second bar represents the negative control nucleic acid group, and the third bar represents the subcutaneous neck injection CpG nucleic acid group.

It is to be understood that the drawings are not required for enablement of the claimed invention.

### Detailed Description of the Invention

The description relates in part, to methods and compositions for promoting weight growth in young non-human animals. This weight growth is induced by the administration of immunostimulatory nucleic acids to such animals. It was not known prior to this description that administration of immunostimulatory nucleic acids induces weight gain in such animals.

The description is also premised on the observation that administration of immunostimulatory nucleic acids to young non-human animals also promotes innate immunity in such animals. These animals include those born as well as those in utero (referred to herein as "fetuses") either by direct administration to the fetus or via the pregnant animal carrying such fetus. It has been previously reported that immunostimulatory nucleic acids are able to induce antigen specific immune responses in young non-human animals. However, it was not known prior to this invention that such administration of immunostimulatory nucleic acids, in the absence of antigen administration, also induces innate immunity in such animals.

Thus, in one aspect, the description provides a method for increasing the rate of growth of feed animals. The method involves administering to a feed animal an immunostimulatory nucleic acid in an effective amount to increase the rate of growth of the feed animal.

The rate of growth is generally measured by weight of the animal, however it is possible to also measure other body dimensions such as height, width, shoulder span, or wing span. One of ordinary skill in the art will be familiar with the methods and parameters for determining growth and rate thereof in agricultural livestock, and any such common standards can be employed in the present description. In some embodiments, more than one parameter may be used to measure growth, while in others, one parameter alone is sufficient.

Similarly, an increase in the rate of growth can be measured by comparison of the value of one or more parameters (preferably, industry standard parameters) in animals receiving an immunostimulatory nucleic acid with animals that receive a control. A control as used herein can be any composition that does not contain an immunostimulatory nucleic acid. Accordingly, the control can be a composition of the suspending buffer or it can be a composition that contain the suspending buffer along with an nucleic acid having no identifiable immunostimulatory motif. As discussed in greater detail below, the immunostimulatory nucleic acid is administered in an amount effective to increase the rate of growth of the feed animal. An effective amount will be that amount that reduces the time required to achieve an acceptable size or weight. The definition of an acceptable size will depend upon the future use of the animal. If the animal is intended for human consumption, then the acceptable size is that minimum size that must be achieved prior to slaughter of the animal, according to industry standards. If the animal is intended for use in an experimental setting, then the acceptable size is that minimum size that must be achieved prior to transport or use in an experimental protocol in order to ensure the viability and health of the animal, again according to industry standards (e.g., see Jackson Laboratories Catalogue, Bar Harbor, ME).

In different embodiments, the description aims to reduce the time required for feed or laboratory animals to achieve an acceptable weight by at least 1 day, at least 2 days, at least 3 days, at least 4 days, at least 5 days, at least 6 days, at least 7 days, at least 2 weeks, at least 3 weeks, at least 4 weeks, at least 2 months, and in some instances at least 6 months, or more. The reduction is time will generally depend on the species of animal and its life span and time to maturity. Time reductions that would be most suitable for feed animal growers can be determined by those of skill in the art, with respect to profitability and suitability of the animal for human consumption. As an example, in embodiments in which the feed animal is a chicken, the effective amount is that amount which reduces the time required for the chicken to reach acceptable size for feed by one day, compared to a chicken that is not administered the immunostimulatory nucleic acid. In other embodiments, the effective amount is that amount which reduces the time by 2 days, 3 days, 4 days, 5 days, 6 days or more, provided that the chicken is considered healthy and suitable for human consumption.

Table 1 indicates the range of average weights per species for some animals slaughtered for human consumption in the United States. The table indicates that generally cattle are slaughtered once they achieve an average weight ranging from 1209 to 1229 lbs., while pigs are slaughtered once they achieve an average weight ranging from 258 to 263 lbs. These figures are derived from industry sources, and similar information can be attained for species of feed animals. Thus, another way of expressing the effective amount is that amount of immunostimulatory nucleic acid that allows, for example, a cow to achieve a weight of roughly 1209 lbs. in one day less, or three days less, or one week less, or two weeks less, or one month less time than it would take a cow not having received an immunostimulatory nucleic acid to achieve that same weight.

**Table 1:**

| **Animal** | **Average Gestation/Incubation Period (Days)** | **Average Ranges of Weight at Slaughter (Pounds)** |
|---|---|---|
| Cow | 284 | Cattle: 1209-1229 |
| | | Calf: 286-302 |
| Deer (White-Tailcd) | 201 | |
| Goat (Domestic) | 151 | |
| Horse | 330 | |
| Pig (Domestic) | 112 | 258-263 |
| Rabbit (Domestic) | 31 | |
| Sheep (Domestic) | 154 | 129-143 |
| Chicken | 21 | Young: 4.9-5.0 |
| | | Mature: 5.2-5.6 |
| Duck | 30 | 6.5-6.6 |
| Goose | 30 | |
| Pigeon | 18 | |
| Turkey | 26 | 25.3-26.6 |

As used herein, a feed animal is an animal that is eaten by humans (i.e., it is used in human food products). Feed animals are generally used as sources of meat for human consumption, but they can also be animals that provide by-products for human consumption, such as eggs and milk. Common examples of feed animals include but are not limited to cows (including calves), pigs, sheep (including lambs), and winged species including chickens, geese, ducks, turkeys, quails, and pigeon. Other less common types of feed animals include deer, goats, horses, rabbits, buffalo, ostrich, and aquatic species such as shell fish (e.g., shrimp, lobster, clams, oyster, mussels, etc.) and octopus.

The description also embraces the use of immunostimulatory nucleic acids for promoting the growth of laboratory animals such as mice and rats. A laboratory animal is an animal that is intended for use in an experimental protocol, and which commonly is deliberately bred to be used as an experimental animal. The ability to increase the rate of growth of such laboratory animals would enable commercial animal colonies to breed animals at a faster rate, leading to a cost savings. Another advantage of growth stimulation in such mice is the ability to identify mutations in stock animals more readily, particularly if such mutations are manifest more definitively at the adult or young adult stage. In addition to mice and rats, the method can also be used to expedite the maturation process of other laboratory animals having a longer life span (and accordingly, a longer maturation period). Thus, as used herein, a laboratory animal can be selected from the group consisting of mice, rats, rabbits, sheep, pigs, monkeys, zebrafish, and dogs, but is not so limited. In preferred embodiments, the laboratory animals are not yet subject to an experimental protocol prior to administration of the immunostimulatory nucleic acid. In other words, the immunostimulatory nucleic acids are administered uniformly to a laboratory animal population (perhaps of a particular species) prior to such animals being selected and used in an experimental procedure.

Some aspects of the description embrace the administration of immunostimulatory nucleic acids to young non-human animals for the purpose of promoting growth and/or innate immunity. A young non-human animal is an animal that is not yet an adult. In some embodiments, the young non-human animal is a neonate (i.e., a newborn). In other embodiments, the young non-human is a fetus.

In some embodiments, the young animal is at an age that represents less than 10% of the average life span of such animal, less than 7% of the average life span of such animal, or less than 5% of the average life span of such animal. As an example, if cows have an average life span of about 15 years (see Table 2), then a young cow would include those that are less than 1.5 years old (i.e., 10%), less than 1.05 years old (i.e., 7%), and less than 9 months old (i.e., 5%). The ages of young non-human animals can similarly be determined with reference to Table 2 below. In other embodiments, the young non-human animal includes animals that are less than 1 day old, less than 1 week old, less than 1 month old, less than 1 year old, and less than 5 years old, depending upon the species of animal (and particularly their average expected life span) and the industry standard in life staging of such species.

For young non-human animals, the immunostimulatory nucleic acids can be administered at a variety of times including within a day of delivery or hatching (i.e., birth), within a week of delivery or hatching, within a month of delivery or hatching, and the like. Administration of the immunostimulatory nucleic acid can begin immediately after birth (for example, within an hour of birth, within two hours of birth, within six hours of birth, or within twelve hours of birth). Administration can be continued as the animal ages or alternatively, it may be discontinued during the aging of the animal. Moreover, administration can take the form of a single bolus or it may be continuous.

**Table 2:**

| **animal** | **gestation (days)** | **average longevity/maximum longevity (years)** |
|---|---|---|
| ass | 365 | 12/35.8 |
| baboon | 187 | 20/35.5 |
| bear (black) | 219 | 18/36.8 |
| bear (grizzly) | 225 | 25/- |
| bear (polar) | 60 | 20/34.7 |
| beaver | 122 | 5/20.5 |
| buffalo (American) | 278 | 15/- |
| camel, bactrian | 406 | 12/29.5 |
| cat (domestic) | 63 | 12/28 |
| chimpanzee | 231 | 20/44.5 |
| chipmunk | 31 | 6/8 |
| cow | 284 | 15/30 |
| deer (white-tailed) | 201 | 8/17.5 |
| dog (domestic) | 61 | 12/20 |
| elephant (African) | 675 | 35/60 |
| elephant (Asian) | 645 | 40/70 |
| elk | 250 | 15/26.5 |
| fox (red) | 52 | 7/14 |
| giraffe | 425 | 10/33.5 |
| goat (domestic) | 151 | 8/18 |
| gorilla | 257 | 20/39.3 |
| guinea pig | 68 | 4/7.5 |
| hippopotamus | 238 | 25/- |
| horse | 330 | 20/46 |
| kangaroo | 42 | 7/- |
| leopard | 98 | 12/19.3 |
| lion | 100 | 15/25 |
| monkey (rhesus) | 164 | 15/- |
| moose | 240 | 12/- |
| mouse (meadow) | 21 | 3/- |
| mouse (dom. white) | 19 | 3/3.5 |
| opossum (American) | 14-17 | 1/- |
| pig (domestic) | 112 | 10/27 |
| puma | 90 | 12/19 |
| rabbit (domestic) | 31 | 5/13 |
| rhinoceros (black) | 450 | 15/- |
| rhinoceros (white) | 480 | 20/- |
| sea lion (California) | 350 | 12/28 |
| sheep (domestic) | 154 | 12/20 |
| squirrel (grey) | 44 | 10/- |
| tiger | 105 | 16/26.3 |
| wolf (maned) | 63 | 5/- |
| zebra (Grant's) | 365 | 15/- |

| | **incubation time (days)** | |
|---|---|---|
| chicken | 21 | |
| duck | 30 | |
| goose | 30 | |
| pigeon | 18 | |
| turkey | 26 | |

Table 2 was taken from The Hutchinson Family Encyclopedia.

In still other embodiments, the immunostimulatory nucleic acids can be administered to animals that are born prematurely. As used herein, an animal that is born prematurely is one which was delivered prior to the average gestation period (or, in the case of bird species, hatched prior to the average incubation period), and preferably, is delivered or hatched prior to the lower end of the normal gestation or incubation range. Table 2 indicates the average gestation and incubation periods for a number of non-human animals. In some important embodiments, the animal that is born prematurely includes those delivered or hatched prior to 95%, 90%, 85%, 80%, 75%, or 50% of the average gestational or incubation period for that animal species. For example, a cow that is born prematurely includes those calves delivered prior to approximately 270 days of gestation (i.e., 95% of the average gestational period of 284 days, as shown in Table 1), or prior to approximately 256 days of gestation (i.e., 90% of the average gestational period), or prior to approximately 241 days of gestation (i.e., 85% of the average gestational period), or prior to approximately 227 days of gestation (i.e., 80% of the average gestational period), or prior to approximately 213 days of gestation (i.e., 75% of the average gestational period), or prior to approximately 142 days of gestation (i.e., 50% of the average gestational period).

The animal may be a multiple delivery animal. A multiple delivery animal is one that is born along with at least one sibling from the same pregnant animal. Such multiple pregnancies can be a common feature of the particular species such as with mice, or alternatively, they may be uncommon as with cattle. The immunostimulatory nucleic acids can be administered to such animals following birth, for example, in order to increase their weight or to promote innate immunity. This is particularly useful since animals born from a multiple pregnancy, particularly those in which such pregnancies are not common, tend to be underdeveloped and of low birth weight

As mentioned above, the description also embraces the administration of immunostimulatory nucleic acids to animals in utero both for the purpose of promoting rate of growth or promoting innate immunity. As used herein, an animal in utero is an animal that is still within the body of its mother, preferably within the uterus and amniotic sac, and which thus has yet to be born. Animals in utero are also referred to herein as fetuses. Such fetuses can be administered the immunostimulatory nucleic acids at any time in the gestation period. In these latter embodiments, the immunostimulatory nucleic acids can be administered to the amniotic sac directly (i.e., trans-placental), or to the pregnant animal carrying the fetus. If administration is to the pregnant animal, administration can take various forms including oral, parenteral (e.g., subcutaneous) and intra-vaginal. As with certain other embodiments, oral administration may be preferred in some instances due to the ease and convenience of administration. Oral administration facilitates the delivery of the nucleic acids along with the animal feed.

Mechanisms of placental transport, as well as agents that effect such transport, are known. Generally, placental transport can occur via endocytosis/exocytosis, lipophilic diffusion, paracellular diffusion, and protein mediated transfer. Endocytosis/exocytosis refers to the uptake of a particular compound from the maternal blood by endocytosis, followed by transport across the cytosol using vesicles, and then release into the fetal circulation. Immunoglobulins are commonly transported to the placenta via this mechanism. Lipophilic diffusion is used to transport hydrophobic molecules that are soluble in plasma membranes such as respiratory gases. Accordingly, an immunostimulatory nucleic acid formulated in a lipid shell or in conjunction with a lipid compound can be transported into the placenta via this latter mechanism. Paracellular diffusion involves the exchange of hydrophilic molecules via water filled channels, and is usually driven by electrochemical imbalances or differences. Compounds transported into the placenta in this manner include small solutes, and ions such as sodium, chloride, and potassium. Protein mediated transport involves the transport of hydrophilic molecules such as ions, amino acids and sugars using specific proteins that bind such molecules. These proteins are located in the maternal and fetal facing plasma membranes. It is possible that immunostimulatory nucleic acids can be transported across the placenta and into the amniotic sac using one or more of these mechanisms depending upon the formulation and conjugation of the nucleic acid.

For aspects of the description directed at promoting innate immunity, it is preferred that the immunostimulatory nucleic acids be administered locally such as in a subcutaneous injection. This is particularly the case in animals which are known to become passively infected with infectious agents in certain bodily sites. An example is chickens which are known to be prone to bacterial infections in their abdominal region. It was surprisingly discovered according to the description that administration of the immunostimulatory nucleic acid to the subcutaneous region in the abdomen provided better protection against a later infection (that entered the body in that area) than did intramuscular injection or subcutaneous administration at a distal site. While intramuscular injection provided systemic immunity, the local subcutaneous injection provided both systemic and local immunity, as evidenced by both better survival outcome and smaller lesion size at the initial site of infection. Moreover, administration of the nucleic acid at the initial site of infection also reduced the number of lesions in other areas of the body, including the pericardium and air sacs of the lungs. Accordingly, animals can be administered immunostimulatory nucleic acids subcutaneously in regions known to be common sites of entry for infectious pathogens, and such administration will improve the innate immunity of such animals, particularly in dealing with future passive infections.

Although not intending to be bound by any particular theory, it is believed that the local innate immunity observed here is due to the recruitment of innate immune system cells such as macrophages and natural killer cells. If immunostimulatory nucleic acids are administered to a site that will be the initial site of infection, then the recruitment of these cells to this site will be useful in eradicating the infection, and preventing or at least reducing its spread throughout the body.

According to some aspects of the description, the immunostimulatory nucleic acids are used to promote or induce innate immunity. As used herein, innate immunity refers to immunity that is not antigen specific and which involves phagocytosis, cytosine release and cytolytic function associated with for example infections. Innate immunity is known to be most effective in the elimination of microbial infections. Enhancement of innate immunity occurs, inter alia, via increased IFN-α production and increased NK cell activity, both of which are effective in the treatment of microbial infections. Innate immunity as used herein is to be distinguished from and is not intended to embrace antibody-dependent cell cytotoxicity.

The immunostimulatory nucleic acids are preferably administered to the animal subjects of the description without the co-administration of an antigen in order to induce innate immunity. The invention is based on the finding that prior administration of an immunostimulatory nucleic acid can result in immunity to later passive and in some cases active exposure to infectious pathogens, such as for example E. coli. The methods described herein therefore do not depend and preferably preclude the co-administration of an antigen to the animal subjects at the time of nucleic acid administration.

In some instances, the non-human animals may be later actively exposed to an antigen for example in the form of a infectious pathogen. For example, the non-human animals may be exposed to the infectious pathogen within a day, within a week, within a month, or within a year of the administration of the immunostimulatory nucleic acid. However, in most instances, it may be preferable not to expose the animals to such agents particularly if they are to used for human consumption, and rather just allow the animals to passively encounter the agent.

The description also provides in another aspect compositions comprising the immunostimulatory nucleic acids of the description formulated for specific routes of administration such as but not limited to oral and subcutaneous administration. For oral administration, the nucleic acids can be formulated as part of an animal feed. As used herein, the term animal feed refers to the food or nutrient source provided to and ingested by animals. In laboratory animals, such animal feed generally takes the form of compressed and dehydrated pellets comprising a mixture of protein, fat, and other nutrients such as vitamins and fiber. Animal feed generally contains these latter components regardless of the species for which it is intended. However, there may be difference between the feed of different species based on different nutritional requirements. Animal feed is commercially available from suppliers such as Greenforest Resources (Ultra Lactozyme-Dry, Bio-Glucanase 10X, BioZyme ABHP, Ultra Acidola Plus, BioActive Yeast, BioLactic Liquid, bio-Adi S.Y.), Shur-Gain, Canada (Rumate products, Optima products, Mash products, Pellet products, Swine feed, Poultry feed, Horse feed), Reco Kraftfutterwerk, Mega Kraftfutterwerk, Emsmühle Papenburg, Wackertrapp, Deuka, Cremer Futtermuchlen, Moehlenkamp GmbH, Broering Dinklage, Bela Muehle, and the like.

The immunostimulatory nucleic acid may be formulated and administered in a lipid based delivery system in an animal feed. The immunostimulatory nucleic acid may be formulated and administered in an alginate delivery system in an animal feed.

In a related aspect, the description embraces compositions comprising the immunostimulatory nucleic acid formulated for subcutaneous administration to a non-human animal. In one important embodiment, the immunostimulatory nucleic acid is formulated in a sustained release vehicle for subcutaneous administration. Such sustained release vehicles are known in the art and include sustained release from microparticles (including nanoparticles) that are injected to the subcutaneous site in a subject. Alternatively, they include subcutaneous patches that degrade and thereby release the immunostimulatory nucleic acid over a period of time.

The administration schedules and doses of the immunostimulatory nucleic acids can vary depending upon the animal, the dose and potency of the immunostimulatory nucleic acid, and the relative risk of infection or the rate at which growth promotion is sought. In some embodiments, the immunostimulatory nucleic acids are administered on a regular basis, such as a daily basis, a weekly basis, or a monthly basis. In some instances, it may not be preferably to administer to the nucleic acid frequently due to the cost of animal handling and the stress that it may cause the animal. Accordingly, in some embodiments, a daily administration is not preferred. However, if the immunostimulatory nucleic acid is administered via a sustained release vehicle, then administration can occur continuously without extra cost or stress.

In embodiments involving chickens, the effective amount in some embodiments will range from 1 *µ*g to 500 *µ*g, preferably from 5 *µ*g to 250 *µ*g, more preferably from 5 *µ*g to 50 *µ*g, and even more preferably from 10 *µ*g to 50 *µ*g, per chicken. As demonstrated in the Examples, administration of 10 *µ*g or 50 *µ*g of CpG immunostimulatory nucleic acids was sufficient to induce a substantial innate immunity to later exposure with E. coli.

The compounds useful according to the description are nucleic acids. The nucleic acids may be double-stranded or single-stranded. Generally, double-stranded molecules may be more stable *in vivo,* while single-stranded molecules may have increased activity. The terms "nucleic acid" and "oligonucleotide" refer to multiple nucleotides (i.e. molecules comprising a sugar (e.g. ribose or deoxyribose) linked to a phosphate group and to an exchangeable organic base, which is either a substituted pyrimidine (e.g. cytosine (C), thymine (T) or uracil (U)) or a substituted purine (e.g. adenine (A) or guanine (G)). As used herein, the terms refer to oligoribonucleotides as well as oligodeoxyribonucleotides. The terms shall also include polynucleosides (i.e. a polynucleotide minus the phosphate) and any other organic base containing polymer. The terms "nucleic acid" and "oligonucleotide" also encompass nucleic acids or oligonucleotides with substitutions or modifications, such as in the bases and/or sugars. For example, they include nucleic acids having backbone sugars which are covalently attached to low molecular weight organic groups other than a hydroxyl group at the 3' position and other than a phosphate group at the 5' position. Thus modified nucleic acids may include a 2'-O-alkylated ribose group. In addition, modified nucleic acids may include sugars such as arabinose instead of ribose. Thus the nucleic acids may be heterogeneous in backbone composition thereby containing any possible combination of polymer units linked together such as peptide- nucleic acids (which have amino acid backbone with nucleic acid bases). In some embodiments, the nucleic acids are homogeneous in backbone composition.

Nucleic acids also include substituted purines and pyrimidines such as C-5 propyne modified bases (Wagner et al., Nature Biotechnology 14:840- 844, 1996). Purines and pyrimidines include but are not limited to adenine, cytosine, guanine, thymine, 5-methylcytosine, 2-aminopurine, 2-amino-6-chloropurine, 2,6-diaminopurine, hypoxanthine, and other naturally and non-naturally occurring nucleobases, substituted and unsubstituted aromatic moieties.

The nucleic acid is a linked polymer of bases or nucleotides. As used herein with respect to linked units of a nucleic acid, linked or linkage means two entities are bound to one another by any physicochemical means. Any linkage known to those of ordinary skill in the art, covalent or non-covalent, is embraced. Such linkages are well known to those of ordinary skill in the art. Natural linkages, which are those ordinarily found in nature connecting the individual units of a nucleic acid , are most common. The individual units of a nucleic acid may be linked, however, by synthetic or modified linkages.

Whenever a nucleic acid is represented by a sequence of letters it will be understood that the nucleotides are in 5'→ 3' order from left to right and that A denotes adenosine, C denotes cytosine, G denotes guanosine, T denotes thymidine, and U denotes uracil unless otherwise noted.

Nucleic acid molecules useful according to the description can be obtained from natural nucleic acid sources (e.g. genomic nuclear or mitochondrial DNA or cDNA), or are synthetic (e.g. produced by oligonucleotide synthesis). Nucleic acids isolated from existing nucleic acid sources are referred to herein as native, natural, or isolated nucleic acids. The nucleic acids useful according to the description may be isolated from any source, including eukaryotic sources, prokaryotic sources, nuclear DNA, mitochondrial DNA, etc. Thus, the term nucleic acid encompasses both synthetic and isolated nucleic acids. The term isolated as used herein refers to a nucleic acid which is substantially free of other nucleic acids, proteins, lipids, carbohydrates or other materials with which it is naturally associated. The nucleic acids can be produced on a large scale in plasmids, (see Sambrook, T., et al, Molecular Cloning: A Laboratory Manual, Cold Spring Harbor laboratory Press, New York, 1989) and separated into smaller pieces or administered whole. After being administered to a subject the plasmid can be degraded into oligonucleotides. One skilled in the art can purify viral, bacterial, eukaryotic, etc. nucleic acids using standard techniques, such as those employing restriction enzymes, exonucleases or endonucleases.

For use in the instant invention, the nucleic acids can be synthesized *de novo* using any of a number of procedures well known in the art. For example, the b-cyanoethyl phosphoramidite method (Beaucage, S.L., and Caruthers, M.H., Tet. Let. 22:1859, 1981); nucleoside H-phosphonate method (Garegg et al., Tet. Let. 27:4051-4054, 1986; Froehler et. al., Nucl. Acid. Res. 14:5399-5407, 1986, ; Garegg et al., Tet. Let. 27:4055-4058, 1986, Gaffney et al., Tet. Let. 29:2619-2622, 1988). These chemistries can be performed by a variety of automated oligonucleotide synthesizers available in the market.

The nucleic acids useful according to the description are immunostimulatory nucleic acids. An immunostimulatory nucleic acid is any nucleic acid, as described above, which is capable of modulating an immune response. A nucleic acid which modulates an immune response is one which produces any form of immune stimulation, including, but not limited to, induction of cytokines, B cell activation, T cell activation, monocyte activation. Accordingly, the immune responses induced by the nucleic acids of the description can be either or both innate and adaptive immune responses. However, since the immunostimulatory nucleic acids of the description are administered in the absence of an antigen (i.e., not co-administered with an antigen), they are intended to function as inducers of innate immunity, at least upon administration. Immunostimulatory nucleic acids include, but are not limited to, CpG nucleic acids, methylated CpG nucleic acids, T-rich nucleic acids, poly G nucleic acids, and nucleic acids having phosphate modified backbones, such as phosphorothioate backbones. Immunostimulatory nucleic acids do not embrace poly I:C and poly A:U RNA, nor immune RNA, in some embodiments.

A CpG nucleic acid or a CpG immunostimulatory nucleic acid as used herein is a nucleic acid containing at least one unmethylated CpG dinucleotide (cytosine-guanine dinucleotide sequence, i.e. CpG DNA or DNA containing an unmethylated 5' cytosine followed by 3' guanosine and linked by a phosphate bond) and activates a component of the immune system. The entire CpG nucleic acid can be unmethylated or portions may be unmethylated but at least the C of the 5' CG 3' must be unmethylated.

Methylated CpG nucleic acids are also immunostimulatory and useful for the purposes of the methods of the description. A methylated CpG nucleic acid is a nucleic acid containing at least one CG dinucleotide in which the C of the CG is methylated and which does not include any unmethylated CG dinucleotides.

In one embodiment, the description provides a CpG nucleic acid or a methylated CpG nucleic acid represented by at least the formula:

5'N₁X₁CGX₂N₂3'

wherein X₁ and X₂ are nucleotides and N is any nucleotide and N₁ and N₂ are nucleic acid sequences composed of from about 0-25 N's each. In some embodiments, X₁ is adenine, guanine, or thymine and X₂ is cytosine, adenine, or thymine. In other embodiments, X₁ is cytosine and/or X₂ is guanine.

In other embodiments, the CpG nucleic acid or methylated CpG nucleic acid is represented by at least the formula:

5'N₁X₁X₂CGX₃X₄N₂3'

wherein X₁, X₂, X₃, and X₄ are nucleotides. In some embodiments, X₁X₂ are nucleotides selected from the group consisting of: GpT, GpG, GpA, ApA, ApT, ApG, CpT, CpA, CpG, TpA, TpT, and TpG; and X₃X₄ are nucleotides selected from the group consisting of: TpT, CpT, ApT, TpG, ApG, CpG, TpC, ApC, CpC, TpA, ApA, and CpA; N is any nucleotide and N₁ and N₂ are nucleic acid sequences composed of from about 0-25 N's each. In some embodiments, X₁X₂ are GpA or GpT and X₃X₄ are TpT. In other embodiments, X₁ or X₂ or both are purines and X₃ or X₄ or both are pyrimidines or X₁X₂ are GpA and X₃ or X₄ or both are pyrimidines.

In some embodiments, N₁ and N₂ of the nucleic acid do not contain a CCGG or CGCG quadmer or more than one CCG or CGG trimer. The effect of a CCGG or CGCG quadmer or more than one CCG or CGG trimer depends in part on the status of the nucleic acid backbone. For instance, if the nucleic acid has a phosphodiester backbone or a chimeric backbone the inclusion of these sequences in the nucleic acid will only have minimal if any affect on the biological activity of the nucleic acid. If the backbone is completely phosphorothioate or significantly phosphorothioate then the inclusion of these sequences may have more influence on the biological activity or the kinetics of the biological activity, but compounds containing these sequences are still useful. In another embodiment, the CpG nucleic acid or the methylated CpG nucleic acid has the sequence 5'TCN₁TX₁X₂CGX₃X₄3'.

In a preferred embodiment, the CpG nucleic acids have a nucleotide sequence selected from the group consisting of TCGTCGTTGTCGTTTTGTCGTT; TCGCGTGCGTTTTGTCGTTTTGACGTT; TCGTCGTTTGTCGTTTTGTCGTT; and ggGGGACGATCGTCgggggG. In this last sequence, the small case G represents residues that are linked by phosphorothioate linkages, and the upper case G represents residues that are linked by phosphodiester linkages.

Examples of CpG nucleic acids include but are not limited to those listed in published PCT application PCT/LJS00/26383 (International Publication Number WO 01/22972), published on April 5, 2001.

A T-rich nucleic acid or T-rich immunostimulatory nucleic acid is a nucleic acid which includes at least one poly T sequence and/or which has a nucleotide composition of greater than 25% T nucleotide residues and which activates a component of the immune system. A nucleic acid having a poly-T sequence includes at least four Ts in a row, such as 5'TTTT3'. Preferably the T-rich nucleic acid includes more than one poly T sequence. In preferred embodiments, the T-rich nucleic acid may have 2, 3, 4, etc. poly T sequences. Other T-rich nucleic acids have a nucleotide composition of greater than 25% T nucleotide residues, but do not necessarily include a poly T sequence. In these T-rich nucleic acids the T nucleotide resides may be separated from one another by other types of nucleotide residues, i.e., G, C, and A. In some embodiments, the T-rich nucleic acids have a nucleotide composition of greater than 30%, 40%, 50%, 60%, 70%, 80%, 90%, and 99%, T nucleotide residues and every integer % in between. Preferably the T-rich nucleic acids have at least one poly T sequence and a nucleotide composition of greater than 25% T nucleotide residues.

In one embodiment, the T-rich nucleic acid is represented by at least the formula:

5'X₁X₂TTTTX₃X₄3'

wherein X₁, X₂,X₃, and X₄ are nucleotides. In one embodiment, X₁X₂ is TT and/or X₃X4 is TT. In another embodiment, X₁X₂ are any one of the following nucleotides TA, TG, TC, AT, AA, AG, AC, CT, CC, CA, CG, GT, GG, GA, and GC; and X₃X₄ are any one of the following nucleotides TA, TG, TC, AT, AA, AG, AC, CT, CC, CA, CG, GT, GG, GA, and GC.

In some embodiments, it is preferred that the T-rich nucleic acid does not contain poly C (CCCC), poly A (AAAA), poly G (GGGG), CpG motifs, or multiple GGs. In other embodiments, the T-rich nucleic acid includes these motifs. Thus in some embodiments, of the description the T-rich nucleic acids include CpG dinucleotides and in other embodiments, the T-rich nucleic acids are free of CpG dinucleotides. The CpG dinucleotides may be methylated or unmethylated.

Examples of T rich nucleic acids that are free of CpG nucleic acids include but are not limited to those described in published PCT application PCT/US00/26383 (International Publication Number WO 01/22972), published on April 5, 2001. This application also describes examples of T rich nucleic acids that include CpG nucleic acids.

Poly G containing nucleic acids are also immunostimulatory. A variety of references, including Pisetsky and Reich, 1993 Mol. Biol. Reports, 18:217-221; Krieger and Herz, 1994, Ann. Rev. Biochem., 63:601-637; Macaya et al., 1993, PNAS, 90:3745-3749; Wyatt et al., 1994, PNAS, 91:1356-1360; Rando and Hogan, 1998, In Applied Antisense Oligonucleotide Technology, ed. Krieg and Stein, p. 335-352; and Kimura et al., 1994, J. Biochem. 116, 991 - 994 also describe the immunostimulatory properties of poly G nucleic acids.

Poly G nucleic acids preferably are nucleic acids having the following formulas:

5'X₁X₂GGGX₃X3'

wherein X₁, X₂, X₃, and X₄ are nucleotides. In preferred embodiments, at least one of X₃ and X₄ are a G. In other embodiments, both of X₃ and X₄ are a G. In yet other embodiments, the preferred formula is 5' GGGNGGG 3', or 5' GGGNGGGNGGG 3' wherein N represents between 0 and 20 nucleotides.

In other embodiments the poly G nucleic acid is free of unmethylated CG dinucleotides, such as, for example, the nucleic acids listed in published PCT application PCT/US00/26383 (International Publication Number WO 01/22972), published on April 5, 2001. This latter application also provides examples of poly G nucleic acids that include at least one unmethylated CG dinucleotide.

Published PCT application PCT/US00/26383 (International Publication Number WO 01/22972), published on April 5, 2001, lists a number of nucleic acids that can be used in the invention. The base designations other than a, c, g and t (or u) (all of which are known in the art) are as follows: i intends inosine; n intends a, c, g, or t, other; d intends a, g, t or u; h intends a, c or t (or u); b intends c, g or t (or u), however if "b" is single and is listed on 5' or 3' end of oligonucleotide, then "b" indicates a biotin moiety attached to that end of the oligonucleotide; q intends 5-methyl-cytosine; m intends a or c; s intends c or g; x, if single and is listed on 5' or 3' end of oligonucleotide, intends a biotin moiety attached to that end of the oligonucleotide; z intends 5-methyl-cytidine; and f intends at least one FITC moiety attached to 5' or 3' end of oligonucleotide.

The backbone modifications listed are as follows: Backbone modifications are abbreviated as follows: S intends phosphorothioate; O intends phosphodiester; SOS intends phosphorothioate and phosphodiester chimeric with phosphodiester in middle; SO intends phosphorothioate and phosphodiester chimeric with phosphodiester on 3' end; OS intends phosphorothioate and phosphodiester chimeric with phosphodiester on 5' end; S2 intends phosphorodithioate; S2O intends phosphorodithioate and phosphodiester chimeric with phosphodiester on 3' end; OS2 intends phosphorodithioate and phosphodiester chimeric with phosphodiester on 5' end; X intends any of the above; and p-ethoxy intends p-ethoxy backbone as in e.g., U.S. Patent No. 6,015,886. In some instances, the nucleic acid may also have a peptide backbone such as for example as in peptide nucleic acids which are known in the art.

Nucleic acids having modified backbones, such as phosphorothioate backbones, also fall within the class of immunostimulatory nucleic acids. U.S. Patents Nos. 5,723,335 and 5,663,153 issued to Hutcherson, et al. and related PCT publication WO95/26204 describe immune stimulation using phosphorothioate oligonucleotide analogues. These patents describe the ability of the phosphorothioate backbone to stimulate an immune response in a non-sequence specific manner.

The immunostimulatory nucleic acid may be any size of at least 2 nucleotides but in some embodiments, are in the range of between 6 and 100 or in some embodiments, between 8 and 35 nucleotides in size. Immunostimulatory nucleic acids can be produced on a large scale in plasmids. These may be administered in plasmid form or alternatively they can be degraded into oligonucleotides.

Palindromic sequence shall mean an inverted repeat (i.e. a sequence such as ABCDEE'D'C'B'A' in which A and A' are bases capable of forming the usual Watson-Crick base pairs and which includes at least 6 nucleotides in the palindrome. *In vivo,* such sequences may form double-stranded structures. In one embodiment, the nucleic acid contains a palindromic sequence. In some embodiments, when the nucleic acid is a CpG nucleic acid, a palindromic sequence used in this context refers to a palindrome in which the CpG is part of the palindrome, and optionally is the center of the palindrome. In another embodiment, the nucleic acid is free of a palindrome. A nucleic acid that is free of a palindrome does not have any regions of 6 nucleotides or greater in length which are palindromic. A nucleic acid that is free of a palindrome can include a region of less than 6 nucleotides which are palindromic.

A stabilized nucleic acid molecule shall mean a nucleic acid molecule that is relatively resistant to *in vivo* degradation (e.g. via an exo- or endo-nuclease). Stabilization can be a function of length or secondary structure. Nucleic acids that are tens to hundreds of kbs long are relatively resistant to *in vivo* degradation. For shorter nucleic acids, secondary structure can stabilize and increase their effect. For example, if the 3' end of an oligonucleotide has self-complementarity to an upstream region, so that it can fold back and form a sort of stem loop structure, then the oligonucleotide becomes stabilized and therefore exhibits more activity.

Some stabilized oligonucleotides of the description have a modified backbone. It has been demonstrated that modification of the oligonucleotide backbone provides enhanced activity of the nucleic acids when administered *in vivo*. Nucleic acids, including at least two phosphorothioate linkages at the 5' end of the oligonucleotide and multiple phosphorothioate linkages at the 3' end, preferably 5, may provide maximal activity and protect the oligonucleotide from degradation by intracellular exo- and endo-nucleases. Other modified oligonucleotides include phosphodiester modified oligonucleotide, combinations of phosphodiester and phosphorothioate oligonucleotide, methylphosphonate, methylphosphorothioate, phosphorodithioate, and combinations thereof. Each of these combinations and their particular effects on immune cells is discussed in more detail in issued U.S. Patents 6,207,646B! and 6,239,116B!, and PCT Published Patent Application PCT/US97/19791 (WO98/18810), published May 7, 1998.

It is believed that these modified oligonucleotides may show more stimulatory activity due to enhanced nuclease resistance, increased cellular uptake, increased protein binding, and/or altered intracellular localization. Both phosphorothioate and phosphodiester nucleic acids are active in immune cells.

Other stabilized oligonucleotides include: nonionic DNA analogs, such as alkyl- and aryl-phosphates (in which the charged phosphonate oxygen is replaced by an alkyl or aryl group), phosphodiester and alkylphosphotriesters, in which the charged oxygen moiety is alkylated. Oligonucleotides which contain diol, such as tetraethyleneglycol or hexaethyleneglycol, at either or both termini have also been shown to be substantially resistant to nuclease degradation.

For use *in vivo,* nucleic acids are preferably relatively resistant to degradation (*e.g*., via endo-and exo-nucleases). Secondary structures, such as stem loops, can stabilize nucleic acids against degradation. Alternatively, nucleic acid stabilization can be accomplished via phosphate backbone modifications. One type of stabilized nucleic acid has at least a partial phosphorothioate modified backbone. Phosphorothioates may be synthesized using automated techniques employing either phosphoramidate or H-phosphonate chemistries. Aryl-and alkyl-phosphonates can be made, *e.g.,* as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165, 1990).

Other sources of nucleic acids useful according to the description include standard viral and bacterial vectors, many of which are commercially available. In its broadest sense, a vector is any nucleic acid material which is ordinarily used to deliver and facilitate the transfer of nucleic acids to cells. The vector as used herein may be an empty vector or a vector carrying a gene which can be expressed. In the case when the vector is carrying a gene the vector generally transports the gene to the target cells with reduced degradation relative to the extent of degradation that would result in the absence of the vector. In this case the vector optionally includes gene expression sequences to enhance expression of the gene in target cells such as immune cells, but it is not required that the gene be expressed in the cell. In preferred embodiments, the gene does not encode an antigen.

In general, vectors include, but are not limited to, plasmids, phagemids, viruses, other vehicles derived from viral or bacterial sources. Viral vectors are one type of vector and include, but are not limited to, nucleic acid sequences from the following viruses: retrovirus, such as Moloney murine leukemia virus, Harvey murine sarcoma virus, murine mammary tumor virus, and Rous sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art. Some viral vectors are based on non-cytopathic eukaryotic viruses in which non-essential genes have been replaced with a nucleic acid to be delivered. Non-cytopathic viruses include retroviruses, the life cycle of which involves reverse transcription of genomic viral RNA into DNA.

Standard protocols for producing empty vectors or vectors carrying genes (including the steps of incorporation of exogenous genetic material into a plasmid, transfection of a packaging cell lined with plasmid, production of recombinant retroviruses by the packaging cell line, collection of viral particles from tissue culture media, and/or infection of the target cells with viral particles) are provided in Kriegler, M., Gene Transfer and Expression, A Laboratory Manual, W.H. Freeman C.O., New York (1990) and Murry, E.J. Ed. Methods in Molecular Biology, vol. 7, Humana Press, Inc., Cliffton, New Jersey (1991).

Other vectors include plasmid vectors. Plasmid vectors have been extensively described in the art and are well-known to those of skill in the art. See e.g., Sambrook et al., Molecular Cloning: A Laboratory Manual, Second Edition, Cold Spring Harbor Laboratory Press, 1989. In the last few years, plasmid vectors have been found to be particularly advantageous for delivering genes to cells *in vivo* because of their inability to replicate within and integrate into a host genome. Some plasmids, however, having a promoter compatible with the host cell, can express a peptide from a gene operatively encoded within the plasmid. Some commonly used plasmids include pBR322, pUC18, pUC19, pcDNA3.1, SV40, and pBlueScript Other plasmids are well-known to those of ordinary skill in the art. Additionally, plasmids may be custom designed using restriction enzymes and ligation reactions to remove and add specific fragments of DNA.

It has recently been discovered that plasmids (empty or gene carrying) can be delivered to the immune system using bacteria. Modified forms of bacteria such as *Salmonella* can be transfected with the plasmid and used as delivery vehicles. The bacterial delivery vehicles can be administered to a host subject orally or by other administration means. The bacteria deliver the plasmid to immune cells, e.g. dendritic cells, probably by passing through the gut barrier. High levels of immune protection have been established using this methodology. Such methods of delivery are useful for the aspects of the description utilizing systemic delivery of nucleic acid.

Other therapeutic agents which can be administered with the nucleic acids of the description are mucosal adjuvants. Mucosal adjuvants are most preferably used when the nucleic acids are administered directly to a mucosal surface (such as occurs during oral administration). The mucosal adjuvants useful according to the description are non-oligonucleotide mucosal adjuvants. A "non-oligonucleotide mucosal adjuvant" as used herein is an adjuvant that is capable of inducing a mucosal immune response in a subject when administered to a mucosal surface in conjunction with an antigen, and it is not nucleic acid in nature. Mucosal adjuvants include but are not limited to Bacterial toxins: e.g., Cholera toxin (CT), CT derivatives including but not limited to CT B subunit (CTB) (Wu et al., 1998, Tochikubo et aL, 1998); CTD53 (Val to Asp) (Fontana et al., 1995); CTK97 (Val to Lys) (Fontana et al., 1995); CTK104 (Tyr to Lys) (Fontana et al., 1995); CTD53/K63 (Val to Asp, Ser to Lys) (Fontana et al., 1995); CTH54 (Arg to His) (Fontana et al., 1995); CTN107 (His to Asn) (Fontana et al., 1995); CTE114 (Ser to Glu) (Fontana et al., 1995); CTE112K (Glu to Lys) (Yamamoto et al., 1997a); CTS61F (Ser to Phe) (Yamamoto et al., 1997a, 1997b); CTS106 (Pro to Lys) (Douce et al., 1997, Fontana et al., 1995); andCTK63 (Ser to Lys) (Douce et al., 1997, Fontana et al., 1995), Zonula occludens toxin, zot, Escherichia coli heat-labile enterotoxin, Labile Toxin (LT), LT derivatives including but not limited to LT B subunit (LTB) (Verweij et al., 1998); LT7K (Arg to Lys) (Komase et al., 1998, Douce et al., 1995); LT61F (Ser to Phe) (Komase et al., 1998); LT112K (Glu to Lys) (Komase et al., 1998); LT118E (Gly to Glu) (Komase et al., 1998); LT146E (Arg to Glu) (Komase et al., 1998); LT192G (Arg to Gly) (Komase et al., 1998); LTK63 (Ser to Lys) (Marchetti et al., 1998, Douce et al., 1997, 1998, Di Tommaso et al., 1996); and LTR72 (Ala to Arg) (Giuliani et al., 1998), Pertussis toxin, PT. (Lycke et al., 1992, Spangler BD, 1992, Freytag and Clemments, 1999, Roberts et al., 1995, Wilson et al., 1995) including PT-9K/129G (Roberts et al., 1995, Cropley et al., 1995); Toxin derivatives (see below) (Holmgren et al., 1993, Verweij et al., 1998, Rappuoli et al., 1995, Freytag and Clements, 1999); Lipid A derivatives (e.g., monophosphoryl lipid A, MPL) (Sasaki et al., 1998, Vancott et al., 1998; Muramyl Dipeptide (MDP) derivatives (Fukushima et al., 1996, Ogawa et al., 1989, Michalek et al., 1983, Morisaki et al., 1983); Bacterial outer membrane proteins (e.g., outer surface protein A (OspA) lipoprotein of *Borrelia burgdorferi, outer membrane protine of Neisseria meningitidis)* (Marinaro et al., 1999, Van de Verg et al., 1996); Oil-in-water emulsions (e.g., MF59) (Barchfield et al., 1999, Verschoor et al., 1999, O'Hagan, 1998); Aluminum salts (Isaka et al., 1998, 1999); and Saponins (e.g., QS21) Aquila Biopharmaceuticals, Inc., Worster, MA) (Sasaki et al., 1998, MacNeal et al., 1998), ISCOMS, MF-59 (a squalene-in-water emulsion stabilized with Span 85 and Tween 80; Chiron Corporation, Emeryville, CA); the Seppic ISA series of Montanide adjuvants (e.g., Montanide ISA 720; AirLiquide, Paris, France); PROVAX (an oil-in-water emulsion containing a stabilizing detergent and a micell-forming agent; IDEC Pharmaceuticals Corporation, San Diego, CA); Syntext Adjuvant Formulation (SAF; Syntex Chemicals, Inc., Boulder, CO); poly[di(carboxylatophenoxy)phosphazene (PCPP polymer, Virus Research Institute, USA) and Leishmania elongation factor (Corixa Corporation, Seattle, WA).

The nucleic acids are delivered in effective amounts. The term effective amount of a nucleic acid refers to the amount necessary or sufficient to realize a desired biologic effect. For example, an effective amount which alone or in combination with other therapeutics, and in single or multiple dosages is effective for increasing the rate of growth in a feed animal and/or increasing innate immunity in non-human animals or fetuses. For instance, an effective amount is that amount which altogether prevents a bacterial infection or at least reduces the number of bacterial lesions in a non-human subject such as a chicken following passive or active bacterial infection. This can be assessed using one of the many known diagnostic assays for infection of feed animals, some of which are used in the Examples. The effective amount may vary depending upon whether a local or a systemic innate immune response is desired, and the route of administration.

Combined with the teachings provided herein, by choosing among the various active compounds and weighing factors such as potency, relative bioavailability, subject body weight, severity of adverse side-effects and preferred mode of administration, an effective regimen can be planned which does not cause substantial toxicity and yet is entirely effective for the purposes outlines herein.

The effective amount for any particular application can vary depending on such factors as the species of non-human animal, the particular nucleic acid being administered (e.g., the number of unmethylated CpG motifs or their locadon in the nucleic acid), the use of simultaneous therapy, or the size of the subject. One of ordinary skill in the art can empirically determine the effective amount of a particular nucleic acid without necessitating undue experimentation.

Subject doses of the compounds described herein typically range from about 0.1 µg to 10 mg per administration, which depending on the application could be given daily, weekly, or monthly and any other amount of time therebetween. More typically mucosal or local doses range from about 5 µg to 5 mg per administration, and most typically from about 5 µg to 1 mg, potentially with 2-4, or more, administrations being spaced hours, days or weeks apart. More typically, immune stimulant doses range from 1 µg to 10 mg per administration, and most typically 5 µg to 1 mg; with daily or weekly administrations. Subject doses of the compounds described herein for parenteral delivery, wherein the compounds are delivered without another therapeutic agent are typically 5 to 10,000 times higher than the effective mucosal dose or for immune stimulant applications, and more typically 10 to 1,000 times higher, and most typically 20 to 100 times higher. More typically parenteral doses for these purposes range from about 5 µg to 5 mg per administration, and most typically from about 5 µg to 1 mg, with 2-4, or more, administrations being spaced hours, days or weeks apart. In some embodiments, however, parenteral doses for these purposes may be used in a range of 5 to 10,000 times higher than the typical doses described above.

In chickens that are administered immunostimulatory nucleic acids either intramuscularly or subcutaneously, the effective amount in some embodiments will range from 1 *µ*g to 500 *µ*g, preferably from 5 *µ*g to 250 *µ*g, more preferably from 5 *µ*g to 50 *µ*g, and even more preferably from 10 *µ*g to 50 *µ*g, per chicken. As demonstrated in the Examples, administration of 10 *µ*g or 50 *µ*g of CpG immunostimulatory nucleic acids was sufficient to induce a substantial innate immunity to later exposure with E. coli.

For use in therapy, an effective amount of the nucleic acid can be administered to a subject by any mode that delivers the nucleic acid to a subject. Administering the pharmaceutical composition of the present description may be accomplished by any means known to the skilled artisan. Some routes of administration include but are not limited to oral, intranasal, intratracheal, inhalation, ocular, vaginal, rectal, parenteral (e.g. intramuscular, intradermal, intravenous or subcutaneous injection) and direct injection.

The formulations of the description are administered in pharmaceutically acceptable solutions, which may routinely contain pharmaceutically acceptable concentrations of salt, buffering agents, preservatives, compatible carriers, adjuvants, and optionally other therapeutic ingredients.

For oral administration, the nucleic acids can be delivered alone without any pharmaceutical carriers or formulated readily by combining the active compound(s) with pharmaceutically acceptable carriers well known in the art. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds of the present description, and with-each other, in a manner such that there is no interaction which would substantially impair the desired Pharmaceutical efficiency.

Such carriers enable the compounds of the description to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers for neutralizing internal acid conditions. Dragee cores may be provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

As mentioned above, if oral administration is used, the nucleic acids are preferably administered along with animal feed.

For administration by inhalation, the compounds for use according to the present description may be conveniently delivered in the form of an aerosol spray, from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.* gelatin for use in an inhaler or insufflator may be formulated containing a powder mix of the compound and a suitable powder base such as lactose or starch.

When it is desirable to deliver the compounds systemically, they may be formulated for parenteral administration by injection, *e.g.,* by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Alternatively, the active compounds may be in powder form for constitution with a suitable vehicle, *e.g.,* sterile pyrogen-free water, before use.

The compounds may also be formulated in rectal or vaginal compositions such as suppositories or retention enemas, *e.g.*, containing conventional suppository bases such as cocoa butter or other glycerides.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions may also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of present methods for drug delivery, see Langer, Science 249:1527-1533, 1990.

The nucleic acids may be administered *per se* (neat) or in the form of a pharmaceutically acceptable salt. When used in medicine the salts should be pharmaceutically acceptable, but non-pharmaceutically acceptable salts may conveniently be used to prepare pbannaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The nucleic acids may be delivered in mixtures in which there may be more than one type of nucleic acid (e.g., a CpG nucleic acid and a T-rich nucleic acid).

A variety of administration routes are available. The particular mode selected will depend, of course, upon the particular nucleic acids selected, the particular risk of infection including the like site of initial infection, and the dosage required for efficacy. The methods of this description, generally speaking, may be practised using any mode of administration that is medically acceptable, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product. Liquid dose units are vials or ampoules. Solid dose units are tablets, capsules and suppositories.

In addition to the formulations described previously, the compounds may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt

In some preferred embodiments, the nucleic acids of the description are administered using a sustained release device, such as those described herein, as well as those known in the art. Other delivery systems can include time-release, or delayed release delivery systems. These systems can avoid repeated administrations of the compounds, increasing convenience to the animal and the breeder (e.g., farmer). Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides.

Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono-, di-, and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the description is contained in a form within a matrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Sustained release devices, their compositions, their method of manufacture and their release kinetics are known in the art and have been described in a variety of U.S. Patents including those to Epic Therapeutics, Inc., Takeda Chemical Industries. Ltd., ALZA Corp., and Alkermes Control Therapeutics, Inc. Reference can be made to U.S. Patents 5,650,173; 5,656,297; 5,679,377; 5,888,533; 5,962,006; 6,110,503; 6,156,331; 6,261,584; 6,265,389; 6,267,981; 6,275,728; 6,268,053; among others.

Sustained release compositions can be applied topically for example as a gel, an ointment, a cream, or a patch (e.g., a transdermal, mucosal, or subcutaneous patch). As an example, sustained release biodegradable particles can be applied to the body surface alone or in the context of an ointment, gel or cream. Topical administration includes administration to a external surface and a mucosal surface. Alternatively, they can be injected (e.g., subcutaneously, intramuscularly, etc.) or implanted. In preferred embodiments, the sustained release devices are biodegradable. In other preferred embodiments, the sustained release devices are adhesive to the surface to which they are applied (e.g., skin or mucosa). The art is familiar with such devices.

The nucleic acid may be directly administered to the subject or may be administered in conjunction with a pharmaceutically acceptable carrier or a delivery vehicle. The nucleic acid and optionally other therapeutic agents may be administered alone (e.g. in saline or buffer) or using any delivery vehicles known in the art. One type of delivery vehicle is referred to herein as a nucleic acid delivery complex. A nucleic acid delivery complex shall mean a nucleic acid molecule associated with (e.g. ionically or covalently bound to; or encapsulated within) a targeting means (e.g. a molecule that results in higher affinity binding to target cell (e.g. dendritic cell surfaces and/or increased cellular uptake by target cells). Examples of nucleic acid delivery complexes include nucleic acids associated with: a sterol (e.g. cholesterol), a lipid (e.g. a cationic lipid, virosome or liposome), or a target cell specific binding agent (e.g. a ligand recognized by target cell specific receptor). Preferred complexes may be sufficiently stable *in vivo* to reduce significant uncoupling prior to internalization by the target cell. However, the complex may be cleavable under appropriate conditions within the cell so that the nucleic acid may be released in a functional form.

The following examples are provided to illustrate specific instances of the practice of the present invention and are not to be construed as limiting the present invention to these examples. As will be apparent to one of ordinary skill in the art, the present invention will find application in a variety of compositions and methods.

### Examples

The following example illustrates the methodology for demonstrating the ability of immunostimulatory nucleic acids to induce both local and systemic innate immunity.
*Introduction:* E. coli causes a variety of disease manifestations (colibacillosis) in poultry, including yolk-sac infection, respiratory tract infection, pericarditis, septicemia, and cellulitis. Cellulitis is the inflammation of the subcutaneous tissue in the abdomen. Pericarditis is inflammation in the pericardium. Airsacculitis is inflammation of the air sacs in the lungs and is akin to pneumonia in a human subject. Polyserositis is regarded as the severest grade of E. coli infection and requires the presence of inflammation of the air sacs, the pericardium and the liver. The objective of this study was to determine the effect of CpG nucleic acids in preventing these manifestations via stimulation of the innate immune system.
*Experimental Protocol:* The experimental protocol for the data presented herein use the well established colibacillosis/cellulitis model. Chickens at 22 days of age were administered CpG immunostimulatory nucleic acid (TCGTCGTTGTCGTTTTGTCGTT) either intramuscularly (in the leg) or subcutaneously in the abdominal area. CpG nucleic acids were administered in either low dose (i.e., 10 *µ*g per bird) or high dose (50 *µ*g per bird). Three days later, the chickens were actively exposed to E. coli by making a 2 cm scratch on the caudal abdominal area and swabbing the scratched area with an E. coli carrying swab. Control chickens did not receive any CpG nucleic acid but they were exposed to E. coli (virulent isolate strain EC317). Each group ranged in number from 20 to 40 birds depending upon the experiment. Chickens were examined for 10 days post E. coli challenge. Clinical scores were evaluated daily and chickens meeting a predetermined criteria were euthanized. Parameters measured included body weight at the time of nucleic acid administration, at the time of E. coli challenge, and at the time of necropsy. Pathological and bacteriological assessments were conducted on all dead or euthanized animals, including all the remaining birds at the termination of the trial on day 10 post-infection with E. coli.

In some experiments, dose titrations were performed using CpG immunostimulatory nucleic acids in absolute doses of 100 *µ*g per bird, 31.6 *µ*g per bird, 10 *µ*g per bird, and 3.16 *µ*g per bird. In addition a control oligonucleotide that lacked any identifiable immunostimulatory motif was administered to control animals. CpG immunostimulatory nucleic acid was also administered at a dose of 31.6 *µ*g per bird to the neck area in other chickens.

### Results:

1. Viability following E. coli challenge: The control group of birds that received no CpG nucleic acid has a survival rate of 15%. In contrast, groups that received CpG nucleic acid by subcutaneous or intramuscular injection had significantly higher survival rates.
   Accordingly, mortality was significantly reduced in all groups receiving CpG nucleic acids compared to control (p<0.01).
2. Size of lesions: The size of the lesions at the site of infection of E. coli was significantly smaller in groups that received CpG nucleic acids by subcutaneous route (p<0.01) as compared to groups that did not receive CpG nucleic acids and those that received CPG by intramuscular injection.
3. CpG injection at a distal site: Injection of CpG nucleic acids intramuscularly or subcutaneously in the neck was less effective at inducing local and systemic innate immunity in chickens as compared to injection of CpG nucleic acids directly to the site of initial infection.

### Conclusions:

These data demonstrate the efficacy of CpG nucleic acid as an immunostimulant in a young non-human animal.

### Equivalents

All terms used herein are to be given their ordinary meaning, as commonly recognized or as recognized in the art to which they belong, unless otherwise specified.

## Claims

1. Use of an immunostimulatory nucleic acid in the manufacture of a composition for preventing infection in a feed animal; wherein said immunostimulatory nucleic acid is to be administered subcutaneously in the abdominal area; and wherein the immunostimulatory nucleic acid is a CpG oligonucleotide or a T-rich oligonucleotide or a poly-G oligonucleotide or a methylated CpG oligonucleotide; wherein the immunostimulatory nucleic acid increases the rate of growth of the feed animal; wherein said immunostimulatory nucleic acid is in an effective amount to increase the rate of growth of the feed animal; and wherein the feed animal is a chicken.

2. A composition comprising an immunostimulatory nucleic acid for use in preventing infection in a feed animal; wherein said immunostimulatory nucleic acid is to be administered subcutaneously in the abdominal area; and wherein the immunostimulatory nucleic acid is a CpG oligonucleotide or a T-rich oligonucleotide or a poly-G oligonucleotide or a methylated CpG oligonucleotide; wherein the immunostimulatory nucleic acid increases the rate of growth of the feed animal; wherein said immunostimulatory nucleic acid is in an effective amount to increase the rate of growth of the feed animal; and wherein the feed animal is a chicken.

3. The use of claim 1 or the composition of claim 2, wherein the immunostimulatory nucleic acid is to be administered at regular intervals.

4. The use of any one of the preceding claims or the composition of any of the preceding claims wherein the immunostimulatory nucleic acid is to be administered to the feed animal immediately after birth or within a day of birth or within a week of birth.

5. The use of any one of the preceding claims or the composition of any of the preceding claims wherein the immunostimulatory nucleic acid is to be administered by injection.

6. The use of any one of the preceding claims or the composition of any of the preceding claims wherein the immunostimulatory nucleic acid is to be administered through a sustained release device.

7. The use of any one of the preceding claims or the composition of any of the preceding claims wherein the immunostimulatory nucleic acid is to be administered in an absolute dosage range of 5 µg to 250 µg

8. The use of any one of the preceding claims or the composition of any of the preceding claims wherein the immunostimulatory nucleic acid has a nucleotide sequence selected from the group consisting of: TCGTCGTTGTCGTTTTGTCGTT; TCGCGTGCGTTTTGTCGTTITGACGTT; TCGTCGTTTGTCGTTTTGTCGTT; and ggGGGACGATCGTCgggggG.

9. The use of any one of the preceding claims or the composition of any one of the preceding claims, wherein the time required for the chicken to reach acceptable size for feed is reduced by one day compared to a chicken that is not administered the immunostimulatory nucleic acid or wherein the time required for the chicken to reach acceptable size for feed is reduced by three days compared to a chicken that is not administered the immunostimulatory nucleic acid.

10. Use of an immunostimulatory nucleic acid in the preparation of a composition for preventing infection in a non-human animal by promoting innate immunity in a non-human animal; wherein said immunostimulatory nucleic acid is to be administered subcutaneously in the abdominal area without the co-administration of an antigen; wherein the immunostimulatory nucleic acid is a CpG oligonucleotide or a T-rich oligonucleotide or a poly-G oligonucleotide or a methylated CpG oligonucleotide; and wherein the non-human animal is at an age which is less than 10% of the average life span of said non-human animal; and wherein the non-human animal is a chicken.

11. A composition comprising an immunostimulatory nucleic acid for use in preventing infection in a non-human animal by promoting innate immunity in a non-human animal; wherein said immunostimulatory nucleic acid is to be administered subcutaneously in the abdominal area without the co-administration of an antigen; wherein the immunostimulatory nucleic acid is a CpG oligonucleotide or a T-rich oligonucleotide or a poly-G oligonucleotide or a methylated CpG oligonucleotide; and wherein the non-human animal is at an age which is less than 10% of the average life span of said non-human animal; and wherein the non-human animal is a chicken.

12. The use of claim 10 or the composition of claim 11 wherein the immunostimulatory nucleic acid is to be administered at regular intervals.

13. The use of any one of claims 10 or 12 or the composition of claim 11 or 12 wherein the immunostimulatory nucleic acid is to be administered to the feed animal immediately after birth or within a day of birth or within a week of birth.

14. The use of any one of claims 10, 12 or 13 or the composition of any one of claims 11 to 13 wherein the immunostimulatory nucleic acid is to be administered at an absolute dose of at least 5 µg.

15. The use of any one of claims 10 and 12 to claim 14 or the composition of any one of claims 11 to 14 wherein the immunostimulatory nucleic acid is to be administered in an absolute dose range of 5 µg to 250 µg.

## Patentansprüche

1. Verwendung einer immunstimulatorischen Nucleinsäure bei der Herstellung einer Zusammensetzung zur Infektionsprävention bei einem Nutztier; wobei die immunstimulatorische Nucleinsäure subcutan in den Abdomenbereich zu verabreichen ist; und wobei die immunstimulatorische Nucleinsäure ein CpG-Oligonucleotid oder ein Oligonucleotid mit hohem T-Anteil oder ein poly-G-Oligonucleotid oder ein methyliertes CpG-Oligonucleotid ist; wobei die immunstimulatorische Nucleinsäure die Wachstumsrate des Nutztieres erhöht; wobei die immunstimulatorische Nucleinsäure in einer Menge vorliegt, die wirksam ist, die Wachstumsrate des Nutztieres zu erhöhen; und wobei das Nutztier ein Huhn ist.

2. Zusammensetzung, umfassend eine immunstimulatorische Nucleinsäure zur Verwendung bei der Infektionsprävention bei einem Nutztier; wobei die immunstimulatorische Nucleinsäure subcutan in den Abdomenbereich zu verabreichen ist; und wobei die immunstimulatorische Nucleinsäure ein CpG-Oligonucleotid oder ein Oligonucleotid mit hohem T-Anteil oder ein Poly-G-Oligonucleotid oder ein methyliertes CpG-Oligonucleotid ist; wobei die immunstimulatorische Nucleinsäure die Wachstumsrate des Nutztieres erhöht; wobei die immunstimulatorische Nucleinsäure in einer Menge vorliegt, die wirksam ist, die Wachstumsrate des Nutztieres zu erhöhen; und wobei das Nutztier ein Huhn ist.

3. Verwendung nach Anspruch 1 oder Zusammensetzung nach Anspruch 2, wobei die immunstimulatorische Nucleinsäure in regelmäßigen Abständen zu verabreichen ist.

4. Verwendung nach einem der vorangehenden Ansprüche oder Zusammensetzung nach einem der vorangehenden Ansprüche, wobei die immunstimulatorische Nucleinsäure dem Nutztier unmittelbar nach der Geburt oder innerhalb eines Tages nach der Geburt oder innerhalb einer Woche nach der Geburt zu verabreichen ist.

5. Verwendung nach einem der vorangehenden Ansprüche oder Zusammensetzung nach einem der vorangehenden Ansprüche, wobei die immunstimulatorische Nucleinsäure mittels Injektion zu verabreichen ist.

6. Verwendung nach einem der vorangehenden Ansprüche oder Zusammensetzung nach einem der vorangehenden Ansprüche, wobei die immunstimulatorische Nucleinsäure mithilfe einer Vorrichtung zur verzögerten Freisetzung zu verabreichen ist.

7. Verwendung nach einem der vorangehenden Ansprüche oder Zusammensetzung nach einem der vorangehenden Ansprüche, wobei die immunstimulatorische Nucleinsäure in einem absoluten Dosierungsbereich von 5 µg bis 250 µg zu verabreichen ist.

8. Verwendung nach einem der vorangehenden Ansprüche oder Zusammensetzung nach einem der vorangehenden Ansprüche, wobei die immunstimulatorische Nucleinsäure eine Nucleotidsequenz aufweist, die ausgewählt ist aus der Gruppe bestehend aus:
TCGTCGTTGTCGTTTTGTCGTT; TCGCGTGCGTTTTOTCCTTTTGACGTT; TCGTCGTTTGTCGTTTTGTCGTT; und ggGGGACGATCGTCgggggG.

9. Verwendung nach einem der vorangehenden Ansprüche oder Zusammensetzung nach einem der vorangehenden Ansprüche, wobei die Zeit, die das Huhn benötigt, um eine annehmbare Größe für den Verzehr zu erreichen, im Verhältnis zu einem Huhn, dem die immunstimulatorische Nucleinsäure nicht verabreicht wurde, um einen Tag reduziert ist oder wobei die die Zeit, die das Huhn benötigt, um eine annehmbare Größe für den Verzehr zu erreichen, im Verhältnis zu einem Huhn, dem die immunstimulatorische Nucleinsäure nicht verabreicht wurde, um drei Tage reduziert ist.

10. Verwendung einer immunstimulatorischen Nucleinsäure bei der Herstellung einer Zusammensetzung zur Prävention von Infektionen bei einem nicht-menschlichen Tier mittels Förderung der angeborenen Immunität bei einem nicht-menschlichen Tier; wobei die immunstimulatorische Nucleinsäure ohne gleichzeitige Verabreichung eines Antigens subcutan in den Abdomenbereich zu verabreichen ist; wobei die immunstimulatorische Nucleinsäure ein CpG-Oligonucleotid oder ein Oligonucleotid mit hohem T-Anteil oder ein Poly-G-Oligonucleotid oder ein methyliertes CpG-Oligonucleotid ist; und wobei das nicht-menschliche Tier ein Alter hat, das weniger als 10% der durchschnittlichen Lebensdauer des nicht-menschlichen Tieres beträgt; und wobei das nicht-menschliche Tier ein Huhn ist.

11. Zusammensetzung, umfassend eine immunstimulatorische Nucleinsäure zur Verwendung bei der Prävention von Infektionen bei einem nicht-menschlichen Tier mittels Förderung der angeborenen Immunität bei einem nicht-menschlichen Tier; wobei die immunstimulatorische Nucleinsäure ohne gleichzeitige Verabreichung eines Antigens subcutan in den Abdomenbereich zu verabreichen ist; wobei die immunstimulatorische Nucleinsäure ein CpG-Ologinucleotid oder ein Oligonucleotid mit hohem T-Anteil oder ein Poly-G-Oligonucleotid oder ein methyliertes CpG-Oligonucleotid ist; und wobei das nicht-menschliche Tier ein Alter hat, das weniger als 10% der durchschnittlichen Lebensdauer des nicht-menschlichen Tieres beträgt; und wobei das nicht-menschliche Tier ein Huhn ist.

12. Verwendung nach Anspruch 10 oder Zusammensetzung nach Anspruch 11, wobei die immunstimulatorische Nucleinsäure in regelmäßigen Abständen zu verabreichen ist.

13. Verwendung nach einem der Ansprüche 10 oder 12 oder Zusammensetzung nach einem der Ansprüche 11 oder 12, wobei die immunstimulatorische Nucleinsäure dem Nutztier unmittelbar nach der Geburt oder innerhalb eines Tages nach der Geburt oder innerhalb einer Woche nach der Geburt zu verabreichen ist.

14. Verwendung nach einem der Ansprüche 10, 12 oder 13 oder Zusammensetzung nach einem der Ansprüche 11 bis 13, wobei die immunstimulatorische Nucleinsäure in einer absoluten Dosis von mindestens 5 µg zu verabreichen ist.

15. Verwendung nach einem der Ansprüche 10 und 12 bis Anspruch 14 oder Zusammensetzung nach einem der Ansprüche 11 bis 14, wobei die immunstimulatorische Nucleinsäure in einem absoluten Dosisbereich von 5 µg bis 250 µg zu verabreichen ist.

## Revendications

1. Utilisation d'un acide nucléique immunostimulant dans la fabrication d'une composition pour prévenir une infection chez un animal de consommation ; où ledit acide nucléique immunostimulant est destiné à être administré par voie sous-cutanée dans le domaine abdominal ; et où l'acide nucléique immunostimulant est un oligonucléotide CpG ou un oligonucléotide riche en T ou un oligonucléotide poly-G ou un oligonucléotide CpG méthylé ; où l'acide nucléique immunostimulant augmente la vitesse de croissance de l'animal de consommation ; où ledit acide nucléique immunostimulant est en une quantité efficace pour augmenter la vitesse de croissance de l'animal de consommation ; et où l'animal de consommation est un poulet.

2. Composition comprenant un acide nucléique immunostimulant destinée à être utilisée pour prévenir une infection chez un animal de consommation ; où ledit acide nucléique immunostimulant est destiné à être administré par voie sous-cutanée dans le domaine abdominal ; et où l'acide nucléique immunostimulant est un oligonucléotide CpG ou un oligonucléotide riche en T ou un oligonucléotide poly-G ou un oligonucléotide CpG méthylé ; où l'acide nucléique immunostimulant augmente la vitesse de croissance de l'animal de consommation ; où ledit acide nucléique immunostimulant est en une quantité efficace pour augmenter la vitesse de croissance de l'animal de consommation ; et où l'animal de consommation est un poulet.

3. Utilisation selon la revendication 1 ou composition selon la revendication 2 où l'acide nucléique immunostimulant est destiné à être administré à intervalles réguliers.

4. Utilisation selon l'une quelconque des revendications précédentes ou composition selon l'une quelconque des revendications précédentes où l'acide nucléique immunostimulant est destiné à être administré à l'animal de consommation immédiatement après la naissance ou dans un intervalle d'un jour par rapport à la naissance ou dans un intervalle d'une semaine p ar rapport à la naissance.

5. Utilisation selon l'une quelconque des revendications précédentes ou composition selon l'une quelconque des revendications précédentes où l'acide nucléique immunostimulant est destiné à être administré par injection.

6. Utilisation selon l'une quelconque des revendications précédentes ou composition selon l'une quelconque des revendications précédentes où l'acide nucléique immunostimulant est destiné à être administré par le biais d'un dispositif à libération prolongée.

7. Utilisation selon l'une quelconque des revendications précédentes ou composition selon l'une quelconque des revendications précédentes où l'acide nucléique immunostimulant est destiné à être administré dans une plage posologique absolue de 5 µg à 250 µg.

8. Utilisation selon l'une quelconque des revendications précédentes ou composition selon l'une quelconque des revendications précédentes où l'acide nucléique immunostimulant a une séquence nucléotidique choisie dans le groupe consistant en : TCGTCGTTGTCGTTTTGTCGTT ; TCGCGTGCGTTTTGTCGTTTTGACGTT ; TCGTCGTTTGTCGTTTTGTCGTT ; et ggGGGACGATCGTCgggggG.

9. Utilisation selon l'une quelconque des revendications précédentes ou composition selon l'une quelconque des revendications précédentes où la durée nécessaire pour que le poulet atteigne une taille acceptable pour la consommation est réduite d'un jour comparée à un poulet auquel l'acide nucléique immunostimulant n'est pas administré ou bien où la durée nécessaire pour que le poulet atteigne une taille acceptable pour la consommation est réduite de trois jours comparée à un poulet auquel l'acide nucléique immunostimulant n'est pas administré.

10. Utilisation d'un acide nucléique immunostimulant dans la préparation d'une composition pour prévenir une infection chez un animal non humain en favorisant l'immunité innée chez un animal non humain ; où ledit acide nucléique immunostimulant est destiné à être administré par voie sous-cutanée dans le domaine abdominal sans la co-administration d'un antigène ; où l'acide nucléique immunostimulant est un oligonucléotide CpG ou un oligonucléotide riche en T ou un oligonucléotide poly-G ou un oligonucléotide CpG méthylé ; et où l'animal non humain est à un âge qui est moins de 10 % de la durée de vie moyenne dudit animal non humain ; et où l'animal non humain est un poulet.

11. Composition comprenant un acide nucléique immunostimulant destinée à être utilisée pour prévenir une infection chez un animal non humain en favorisant l'immunité innée chez un animal non humain ; où ledit acide nucléique immunostimulant est destiné à être administré par voie sous-cutanée dans le domaine abdominal sans la co-administration d'un antigène ; où l'acide nucléique immunostimulant est un oligonucléotide CpG ou un oligonucléotide riche en T ou un oligonucléotide poly-G ou un oligonucléotide CpG méthylé ; et où l'animal non humain est à un âge qui est moins de 10 % de la durée de vie moyenne dudit animal non humain ; et où l'animal non humain est un poulet.

12. Utilisation selon la revendication 10 ou composition selon la revendication 11 où l'acide nucléique immunostimulant est destiné à être administré à intervalles réguliers.

13. Utilisation selon l'une quelconque des revendications 10 ou 12 ou composition selon la revendication 11 ou 12 où l'acide nucléique immunostimulant est destiné à être administré à l'animal de consommation immédiatement après la naissance ou dans un intervalle d'un jour par rapport à la naissance ou dans un intervalle d'une semaine par rapport à la naissance.

14. Utilisation selon l'une quelconque des revendications 10, 12 ou 13 ou composition selon l'une quelconque des revendications 11 à 13 où l'acide nucléique immunostimulant est destiné à être administré à une dose absolue d'au moins 5 µg.

15. Utilisation selon l'une quelconque des revendications 10 et 12 à 14 ou composition selon l'une quelconque des revendications 11 à 14 où l'acide nucléique immunostimulant est destiné à être administré dans une plage posologique absolue de 5 µg à 250 µg.
